# EUROPÄISCHE PATENTSCHRIFT

(11) **EP 4 175 542 B1**
(45) Veröffentlichungstag und Bekanntmachung des Hinweises auf die Patenterteilung: **01.05.2024**
(21) Anmeldenummer: 21740434.2
(22) Anmeldetag: 28.06.2021
(51) Int. Cl.: A61B 5/08, A61B 5/097, A61M 16/08, A61M 16/00, A61M 16/20

(54) **ERMITTLUNGSVORRICHTUNG, MEDIZINGERÄT, COMPUTERPROGRAMMPRODUKT, SPEICHERMITTEL UND VERFAHREN ZUM ERMITTELN EINER KOHLENSTOFFDIOXIDKONZENTRATION IN MESSGAS**
DETERMINATION DEVICE, MEDICAL APPARATUS, COMPUTER PROGRAM PRODUCT, STORAGE MEANS AND METHOD FOR DETERMINING A CARBON DIOXIDE CONCENTRATION IN MEASUREMENT GAS
DISPOSITIF DE DÉTERMINATION, APPAREIL MÉDICAL, PRODUIT DE PROGRAMME INFORMATIQUE, MOYEN DE STOCKAGE ET PROCÉDÉ DE DÉTERMINATION D'UNE CONCENTRATION DE DIOXYDE DE CARBONE DANS UN GAZ DE MESURE

(30) Priorität: 03.07.2020 DE 102020117607
(43) Veröffentlichungstag der Anmeldung: 10.05.2023
(73) Patentinhaber: Drägerwerk AG & Co. KGaA, 23558 Lübeck (DE)
(72) Erfinder: HANSMANN, Hans-Ullrich, 23558 Lübeck (DE)
(86) Internationale Anmeldenummer: PCT/EP2021/067623
(87) Internationale Veröffentlichungsnummer: WO 2022/002816

(56) Entgegenhaltungen:
- WO-A1-2019/072605
- US-A- 5 873 361
- US-A1- 2008 041 172
- US-A1- 2012 215 125
- US-B1- 7 556 039

## Beschreibung

Die vorliegende Erfindung betrifft ein Verfahren zum Ermitteln einer Kohlenstoffdioxidkonzentration in Messgas, insbesondere aus und/oder in einem Medizingerät. Die Erfindung betrifft ferner ein Computerprogrammprodukt, das Befehle umfasst, um ein solches Verfahren durchzuführen, und ein Speichermittel, auf dem ein solches Computerprogrammprodukt gespeichert ist. Darüber hinaus betrifft die Erfindung eine Ermittlungsvorrichtung, ein Medizingerät und eine Einstelleinheit zum Ermitteln der Kohlenstoffdioxidkonzentration.

### STAND DER TECHNIK

Kohlenstoffdioxid ist bei der Beatmung einer Person durch ein Beatmungsgerät einer der wichtigsten Parameter zur Beurteilung der Beatmungseffizienz. Eine präzise und zuverlässige Überwachung der Kohlenstoffdioxidkonzentration ist während der Beatmung deshalb von entscheidender Bedeutung.

Zum Ermitteln der Kohlenstoffdioxidkonzentration kommen verschiedene physikalische und/oder chemische Verfahren in Betracht. Beispielsweise kann die Kohlenstoffdioxidkonzentration mit Hilfe von Infrarotsensoren, elektrochemischen Sensoren, einem colorimetrischen Verfahren, oder auch mit Hilfe von Massenspektrometern erfasst werden. Einige dieser Verfahren weisen einen komplexen Messaufbau auf, sind dadurch entsprechend teuer und/oder für eine kontinuierliche Erfassung der Kohlenstoffdioxidkonzentration nicht geeignet.

Weiterhin ist ein System bekannt, bei welchem mittels der Wärmeleitung eines Messgases bzw. einer Gasprobe an einer Sensoreinheit auf die

Kohlenstoffdioxidkonzentration im Messgas geschlossen werden kann. Für die Ermittlung der Kohlenstoffdioxidkonzentration wird beispielsweise eine Sensoreinheit dicht neben einem sogenannten Mainstream bzw. einer Hauptleitung mittels Diffusion mit Inspirationsgas sowie Exspirationsgas begast. Ein solches System ist aus der deutschen Patentanmeldung DE 10 2010 047 159 A1 bekannt. Dort wird ferner eine hydrophobe Sperre gegen eine kondensierende Feuchte vorgeschlagen. Problematisch bei diesem System ist es, dass Quereinflüsse über Gasparameter wie die Messgastemperatur und/oder die Messgasfeuchte synchron zu den Atemphasen, also der Inspiration und der Exspiration, aufgrund der fehlenden Selektivität in der Sensoreinheit zu einer unzureichend genauen Ermittlung der Kohlenstoffdioxidkonzentration im Messgas führen. Mit anderen Worten, die wechselnde Feuchte durch Inspiration bzw. inspiratorische Phase und Exspiration bzw. exspiratorischen Phase führt je nach Belegung der hydrophoben Sperre zu einer wechselnden Feuchte am Sensor. Dies kann zu veränderten Messwerten und einer entsprechenden Messungenauigkeit sowie zu einer teilweise bis vollständigen Gassperre, durch welche die gewünschte Messung nicht weiter durchgeführt werden kann, führen.

Darüber hinaus sind Messanordnungen bekannt, bei welchen Messgas mittels Pumpe über eine Abzweigungsleitung aus der Hauptleitung zu einer geeigneten Sensoreinheit abgesaugt bzw. abgezweigt wird, um mittels der Sensoreinheit die Kohlenstoffdioxidkonzentration zu ermitteln. Nachteilig bei den bekannten Lösungen dieser Art ist es, dass der durch die Pumpe erzeugte Volumenstrom des Messgases in der Abzweigungsleitung zu einer Beeinflussung des Messsignals führt. Durch den wechselnden Atemwegsdruck am Patienten entsteht eine unterschiedliche Druckdifferenz über der Abzweigungsleitung. Dadurch entsteht ein wechselnder Volumenstrom, der ein atemphasensynchrones Messsignal erzeugt. Dadurch ist es kaum möglich, den für die Ermittlung der Kohlenstoffdioxidkonzentration erforderlichen Kohlenstoffdioxidunterschied zwischen den beiden Atemphasen ausreichend exakt zu ermitteln. Zusätzlich wird durch den wechselnden Volumenstrom der zeitlich korrekte Zusammenhang beider Phasen gestört. Auch das Dokument US 5873361 A offenbart relevanten Stand der Technik.

### OFFENBARUNG DER ERFINDUNG

Aufgabe der vorliegenden Erfindung ist es, der voranstehend beschriebenen Problematik zumindest teilweise Rechnung zu tragen. Insbesondere ist es Aufgabe der vorliegenden Erfindung, eine Vorrichtung und ein Verfahren zum möglichst einfachen, kostengünstigen und genauen Ermitteln der Kohlenstoffdioxidkonzentration in Messgas aus einem gattungsgemäßen Medizingerät zu schaffen.

Die voranstehende Aufgabe wird durch die Patentansprüche gelöst. Insbesondere wird die voranstehende Aufgabe durch das Verfahren gemäß Anspruch 1, die Ermittlungsvorrichtung gemäß Anspruch 8, das Computerprogrammprodukt gemäß Anspruch 14 sowie das Speichermittel gemäß Anspruch 15 gelöst. Weitere Vorteile der Erfindung ergeben sich aus den Unteransprüchen, der Beschreibung und den Figuren. Dabei gelten Merkmale, die im Zusammenhang mit dem Verfahren beschrieben sind, selbstverständlich auch im Zusammenhang mit der erfindungsgemäßen Ermittlungsvorrichtung, dem erfindungsgemäßen Medizingerät, der erfindungsgemäßen Einstelleinheit, dem erfindungsgemäßen Computerprogrammprodukt, dem erfindungsgemäßen Speichermittel und jeweils umgekehrt, sodass bzgl. der Offenbarung zu den einzelnen Erfindungsaspekten stets wechselseitig Bezug genommen wird und/oder werden kann.

Gemäß einem ersten Aspekt der vorliegenden Erfindung wird ein Verfahren zum Ermitteln einer Kohlenstoffdioxidkonzentration in Messgas laut Anspruch 1 zur Verfügung gestellt.

Es wird demnach vorgeschlagen, die Fluidfördereinheit, beispielsweise in Form einer Absaugepumpe, mit dem Vorwissen des Atemwegsdrucks derart anzusteuern, dass der Volumenstrom während den unterschiedlichen Atemphasen bzw. während der inspiratorischen Phase, insbesondere der inspiratorischen Phase einer Beatmung durch das Medizingerät, und während der exspiratorischen Phase, insbesondere der exspiratorischen Phase einer Beatmung durch das Medizingerät, möglichst konstant bleibt oder dass die Änderungen des Volumenstroms während den unterschiedlichen Atemphasen zumindest möglichst klein gegenüber den Kohlenstoffdioxidkonzentrationswechseln zwischen der Kohlenstoffdioxidkonzentration in der exspiratorischen Phase und der kohlenstoffdioxidfreien oder im Wesentlichen kohlenstoffdioxidfreien inspiratorischen Phase sind oder werden.

Unter dem Abzweigen des Messgases aus der Hauptleitung des Medizingerätes während der inspiratorischen Phase und während der exspiratorischen Phase ist hierbei zu verstehen, dass das Messgas von Beginn der inspiratorischen Phase an bis zum Ende der exspiratorischen Phase und anschließend insbesondere ohne Unterbrechung grundsätzlich beliebig häufig erneut von Beginn einer weiteren inspiratorischen Phase an bis hin zum Ende einer weiteren exspiratorischen Phase durchgehend abgezweigt wird. Darunter, dass die Fluidfördereinheit zum Erzeugen eines während der inspiratorischen Phase und der exspiratorischen Phase gleichmäßigen Volumenstroms und/oder Gasdrucks des Messgases in der Abzweigungsleitung zur Sensoreinheit adaptiv eingestellt wird kann entsprechend verstanden werden, dass die Fluidfördereinheit unter Berücksichtigung des aktuellen Atemwegsdrucks in der Hauptleitung derart adaptiv eingestellt wird, dass ein Volumenstrom und/oder ein Gasdruck des Messgases in der Abzweigungsleitung zur Sensoreinheit erzeugt wird, der von der inspiratorischen Phase an über die exspiratorischen Phase und weitere mögliche, anschließende inspiratorischen Phasen und exspiratorischen Phase möglichst durchgehend gleich bleibt und/oder möglichst nur geringe Änderungen aufweist. Unter der inspiratorischen Phase der Person kann ein Einatemvorgang durch die Person mit oder ohne zumindest teilweise Unterstützung durch das Medizingerät verstanden werden. Unter der exspiratorischen Phase der Person kann ein Ausatemvorgang durch die Person mit oder ohne zumindest teilweise Unterstützung durch das Medizingerät verstanden werden.

Im Rahmen der vorliegenden Erfindung wurde zunächst erkannt, dass mit einem einfachen Messaufbau unter Verwendung der Abzweigungsleitung, der Sensoreinheit und der Fluidfördereinheit grundsätzlich bereits relativ kleine Volumenströme in der Abzweigungsleitung für die gewünschte Ermittlung der Kohlenstoffdioxidkonzentration ausreichen. Üblicherweise werden für sogenannte absaugende Kohlenstoffdioxidmessungen im Messgas Absaugevolumenströme von in der Regel 100 ml/min bis 200 ml/min verwendet bzw. eingestellt. Solche Volumenströme sind aufgrund der verwendeten Komponenten wie Filter, Schläuche und Entfeuchtungsvorrichtungen nötig. Vorliegend wurde nun erkannt, dass einfache, optional als Einmal-Nutzen aufgebaute Absaugeeinheiten nicht nur mit deutlich kleineren Volumenströmen in einem Bereich zwischen beispielsweise 40 ml/min und 60 ml/min sowie mit relativ kleinen Gasdrücken in einem Bereich zwischen beispielsweise 25 mbar und 35 mbar auskommen, sondern dabei auch noch ausreichend Messgas zur Sensoreinheit für die gewünschte Ermittlung der Kohlenstoffdioxidkonzentration im Messgas fördern.

Durch den kleinen Gasdruck und/oder den kleinen Volumenstrom entsteht jedoch eine relativ hohe Abhängigkeit zu den Atemwegsdrücken des Patienten und/oder des Medizingerätes. Der Volumenstrom und die Gasdrücke während der Absaugung variieren mit den Gasdrücken in den Atemphasen des durch das Medizingerät beatmeten Patienten und/oder des Medizingerätes. Um diesen Einfluss zu verhindern oder zumindest zu reduzieren, wird die Fluidfördereinheit mit dem Wissen des Atemwegsdrucks bzw. eines entsprechenden Patientendrucks so angesteuert, dass der Volumenstrom in der Abzweigungsleitung und/oder der Gasdruck in der Abzweigungsleitung möglichst konstant bleiben. Auf eine zusätzliche Volumenstrom-Messung soll hierbei möglichst verzichtet werden.

Der Atemwegsdruck wechselt bei einer üblichen Beatmung einer Person von beispielsweise kontinuierlich zwischen ca. 25 mbar während der inspiratorischen Phase und beispielsweise ca. 5 mbar während der exspiratorischen Phase. Soll nun Messgas mit beispielsweise 50 ml/min durch die Abzweigungsleitung zur Sensoreinheit gefördert werden, wird während der inspiratorischen Phase nur eine geringe Unterstützung durch die Fluidfördereinheit benötigt.

Während der exspiratorischen Phase ist hingegen eine deutlich höhere Absaugleistung erforderlich, da die Druckdifferenz gegenüber der Umgebung nur noch 1/5 beträgt. Für einen Gasfluss von beispielsweise 50 ml/min sind ca. 30 mbar erforderlich. Hat der Patient einen Atemwegsdruck von beispielsweise 25 mbar und/oder ist wenigstens teilweise durch das Medizingerät ein solcher Atemwegsdruck eingestellt, muss die Fluidfördereinheit während der inspiratorischen Phase lediglich 5 mbar zusätzlich erzeugen, um den angestrebten Volumenstrom zu erzielen. Während der exspiratorischen Phase mit beispielsweise nur 5 mbar Atemwegsdruck werden zusätzliche 25 mbar durch die Fluidfördereinheit benötigt, um den gleichen Volumenstrom zu erzielen.

Die Fluidfördereinheit, insbesondere ein Pumpenunterdruck einer als Pumpe ausgestalteten Fluidfördereinheit, kann nun so eingestellt werden, dass die Summe aus Atemwegsdruck und Pumpendruck bzw. dem durch die Fluidfördereinheit erzeugten Druck zusammen 30 mbar ergibt.

Ein solcher Ablauf ist in der nachfolgenden Tabelle dargestellt.

| **Phase** | **Dauer [s]** | **Zeitpunkt Phasenbeginn [s]** | **PAW [mbar]** | **Absaugdruck [mbar]** | **Summe Druck über Abzweigungsleitung** | **Volumenstrom in Abzweigungsleitung** |
|---|---|---|---|---|---|---|
| Beginn Inspiration | 0,3 | T | 5 → 25 | 25 → 5 | 30 | 50 |
| Inspiration | 2,7 | T + 0,3 | 25 | 5 | 30 | 50 |
| Beginn Exspiration | 0,3 | T + 3 | 25 → 5 | 5 → 25 | 30 | 50 |
| Exspiration | 2,7 | T + 3,3 | 5 | 25 | 30 | 50 |
| Beginn weitere Inspiration | 0,3 | T + 6 | 5 → 25 | 25 → 5 | 30 | 50 |

Bei bekannten, absaugenden Ermittlungsvorrichtungen werden aufgrund der dort verwendeten Filter, Wasserfälle, Abzweigungsleitungen und erheblich höheren Gasflüsse bzw. Volumenströme von beispielsweise 200 ml/min im Vergleich zur vorgeschlagenen Ermittlungsvorrichtung und/oder Messanordnung relativ hohe Druckabfälle erzeugt, sodass auch die Druckdifferenz für die Leistung der Fluidfördereinheit in einem deutlich höheren Bereich zwischen beispielsweise 150 mbar bis 200 mbar liegt. Hier ist der zusätzliche Druckunterschied durch den Atemwegsdruck untergeordnet, die Fluidfördereinheit, insbesondere in Form einer Pumpe, kann mit einer konstanten Leistung laufen und einen mittleren Gasfluss erzeugen. Bei derartigen Systemen musste man deshalb bislang keine Überlegungen zu einer erfindungsgemäßen Ansteuerung der Fluidfördereinheit bzw. zum adaptiven Einstellen der Fluidfördereinheit unter Berücksichtigung des Atemwegsdrucks anstellen.

Zum Ermitteln einer Kohlenstoffdioxidkonzentration im Messgas wird in der Sensoreinheit insbesondere die Wärmeleitfähigkeit des Exspirationsgases gemessen. Die Messung erfolgt durch ein mikrostrukturiertes Heizelement auf einer dünnen Membran der Sensoreinheit. Neben dem Heizelement befindet sich eine thermophile Anordnung, die eine Übertemperatur des Gases nahe dem Heizelement in Bezug auf einen Siliziumrahmen der Membran misst. Die Ermittlung der Kohlenstoffdioxidkonzentration mittels Wärmeleitfähigkeit des Messgases ist im Stand der Technik grundsätzlich bekannt. Weitere Details können beispielsweise der deutschen Patentanmeldung DE 10 2010 047 159 A1 entnommen werden. Im Rahmen der vorliegenden Erfindung wurden jedoch die Wichtigkeit eines konstanten Volumenstroms des Messgases durch die Abzweigungsleitung für die Ermittlung der Kohlenstoffdioxidkonzentration im Messgas unter Berücksichtigung der Wärmeleitfähigkeit des Messgases erkannt und daraufhin das vorgeschlagene Verfahren entwickelt. Die Sensoreinheit umfasst folglich vorzugsweise einen Wärmeleitungssensor zum Messen der Wärmeleitfähigkeit und/oder der Wärmeleitung des Messgases, also der Wärmeleitfähigkeit des abgezweigten Teils des Inspirationsgases sowie des abgezweigten Teils des Exspirationsgases. Anhand der gemessenen Wärmeleitfähigkeit wird anschließend die Kohlenstoffdioxidkonzentration ermittelt. Darunter, dass die Kohlenstoffdioxidkonzentration mittels der Sensoreinheit ermittelt wird kann mithin verstanden werden, dass die Kohlenstoffdioxidkonzentration anhand verschiedener Messungen und Berechnungen ermittelt wird und hierbei die Sensoreinheit verwendet wird, oder anders ausgedrückt, dass die Kohlenstoffdioxidkonzentration im Messgas anhand einer durch die Sensoreinheit gemessene Wärmeleitfähigkeit des Messgases ermittelt wird. Mittels der Sensoreinheit können die Kohlenstoffdioxidunterschiede ermittelt werden und anhand der Messwerte wird die Kohlenstoffdioxidkonzentration berechnet und/oder anhand von beispielsweise einer Look-Up-Tabelle ermittelt. Wie vorstehend bereits erwähnt, umfasst das Messgas deshalb vorzugsweise Inspirationsgas und Exspirationsgas. Demnach kann durch die Kohlenstoffdioxiddifferenz zwischen dem Inspirationsgas und dem Exspirationsgas die relative Kohlenstoffdioxidkonzentration im Exspirationsgas ermittelt werden.

Das Verfahren zum Ermitteln der Kohlenstoffdioxidkonzentration wird insbesondere als Verfahren zum Ermitteln der Kohlenstoffdioxidkonzentration während einer Beatmung der am Medizingerät angeschlossenen Person durch das Medizingerät und/oder zumindest während eines Betriebs des Medizingerätes durchgeführt. Demnach kann das adaptive Einstellen der Fluidfördereinheit zumindest zeitweise ohne aktuelle Informationen über den vorliegenden Atemwegsdruck, beispielsweise in Form eines Notprogramms und/oder eines Übergangsprogramms, durchgeführt werden. Das Verfahren kann als ein Verfahren zum Ermitteln einer Kohlenstoffdioxidkonzentration in Messgas aus einer Hauptleitung bzw. aus dem sogenannten Mainstream eines Medizingerätes, insbesondere eines Beatmungsgerätes, durchgeführt werden. Unter dem adaptiven Einstellen der Fluidfördereinheit kann insbesondere ein kontinuierliches Einstellen und/oder Verstellen der Fluidfördereinheit abhängig vom aktuellen Atemwegsdruck in der Hauptleitung verstanden werden. D.h., die Fluidfördereinheit wird abhängig vom aktuellen Atemwegsdruck in der Hauptleitung unterschiedlich einstellt und/oder angesteuert.

Das adaptive Einstellen der Fluidfördereinheit wird insbesondere durch eine geeignete Einstelleinheit, beispielsweise in Form eines Steuergeräts des Medizingerätes, durchgeführt. Unter dem Messgas kann eine an der Sensoreinheit zu messende Gasprobe verstanden werden. Das Messgas entspricht während der inspiratorischen Phase einem Teil des Inspirationsgases und während der exspiratorischen Phase einem Teil des Exspirationsgases. Mit anderen Worten, ein Teil des Inspirationsgases wird während der inspiratorischen Phase als Messgas abgezweigt und ein Teil des Exspirationsgases wird während der exspiratorischen Phase ebenfalls als Messgas abgezweigt.

Unter dem Fördern des Messgases kann ein Absaugen des Messgases aus der Hauptleitung in die Abzweigungsleitung zur Sensoreinheit hin verstanden werden. Die Fluidfördereinheit ist in diesem Fall insbesondere als Pumpe und/oder Absaugpumpe konfiguriert. Die dargestellten Verfahrensschritte müssen nicht in der angegebenen Reihenfolge durchgeführt werden. Vielmehr können die Verfahrensschritte zumindest teilweise gleichzeitig durchgeführt werden. Unter einem gleichmäßigen Volumenstrom kann vorliegend ein Volumenstrom verstanden werden, dessen Wert sich über die Zeit von wenigstens einer inspiratorischen Phase und einer daran anschließenden exspiratorischen Phase um weniger als 20%, insbesondere um weniger als 10%, verändert.

Der Atemwegsdruck entspricht während der inspiratorischen Phase dem Gasdruck des Inspirationsgases in der Hauptleitung und während der exspiratorischen Phase dem Gasdruck des Exspirationsgases in der Hauptleitung. Unter dem Atemwegsdruck kann folglich auch der Gasdruck, insbesondere der Gasdruck des Inspirationsgases und der Gasdruck des Exspirationsgases, in der Hauptleitung verstanden werden. Der Atemwegsdruck kann als tatsächlicher Atemwegsdruck gemessen und/oder als eingestellter Atemwegsdruck ermittelt bzw. verwendet werden.

Bei dem erfindungsgemäßen Verfahren wird der Atemwegsdruck in der Hauptleitung durch einen Atemwegsdruck-Sensor gemessen und die Fluidfördereinheit, zum Erzeugen eines gleichmäßigen Volumenstroms und/oder Gasdrucks des Messgases in der Abzweigungsleitung zur Sensoreinheit, unter Verwendung des gemessenen Atemwegsdrucks in der Hauptleitung adaptiv eingestellt wird. D. h., die Fluidfördereinheit wird unter Verwendung des tatsächlich gemessenen Atemwegsdrucks eingestellt. Damit kann die Kohlenstoffdioxidkonzentration im Messgas besonders genau ermittelt werden. Hierbei kann die Hauptleitung einen Inspirationsgasleitungsabschnitt zum Leiten des Inspirationsgases, insbesondere von ausschließlich dem Inspirationsgas, und einen Gesamtgasleitungsabschnitt zum Leiten des Inspirationsgases sowie des Exspirationsgases aufweisen, wobei der Atemwegsdruck im und/oder am Gesamtgasleitungsabschnitt gemessen wird.

Im Rahmen der Erfindung wird ferner ein Verfahren zum Ermitteln der Kohlenstoffdioxidkonzentration im Messgas während einer druckgeführten Beatmung der Person durch das Medizingerät vorgeschlagen, wobei die Fluidfördereinheit, zum Erzeugen eines gleichmäßigen Volumenstroms und/oder eines gleichmäßigen Gasdrucks des Messgases in der Abzweigungsleitung zur Sensoreinheit, unter Verwendung eines durch die druckgeführte Beatmung der Person eingestellten Atemwegsdrucks in der Hauptleitung, adaptiv eingestellt wird. Durch die druckgeführte Beatmung der Person kann auf einen wie vorstehend erwähnten Atemwegsdruck-Sensor verzichtet werden. Der für das adaptive Einstellen der Fluidfördereinheit benötigte Atemwegsdruck kann beispielsweise direkt aus einem Steuergerät des Medizingerätes ausgelesen und verwendet werden. Die Fluidfördereinheit kann demnach unter Verwendung des für den Betrieb des Medizingerätes eingestellten Atemwegsdrucks für die druckgeführte Beatmung adaptiv eingestellt werden. Ferner ist es möglich, dass bei einem erfindungsgemäßen Verfahren zum Ermitteln der Kohlenstoffdioxidkonzentration im Messgas während einer volumengeführten Beatmung der Person durch das Medizingerät die Fluidfördereinheit, zum Erzeugen eines gleichmäßigen Volumenstroms und/oder Gasdrucks des Messgases in der Abzweigungsleitung zur Sensoreinheit, unter Verwendung eines aus der volumengeführten Beatmung der Person resultierenden Atemwegsdrucks in der Hauptleitung adaptiv eingestellt wird.

Bei dem Verfahren gemäß der vorliegenden Erfindung weist die Fluidfördereinheit zum Fördern des Messgases aus der Hauptleitung durch die Abzweigungsleitung zur Sensoreinheit eine Piezopumpe auf und eine Betriebsspannung der Piezopumpe, zum Erzeugen eines gleichmäßigen Volumenstroms und/oder Gasdrucks des Messgases in der Abzweigungsleitung zur Sensoreinheit, wird unter Berücksichtigung des Atemwegsdrucks sowie unter Verwendung einer Look-Up-Tabelle adaptiv eingestellt.

Unter Verwendung der Piezopumpe und einer zugehörigen Look-Up-Tabelle, insbesondere eines gattungsgemäßen Kennliniendiagramms mit charakteristischen Kennlinien, kann die Fluidfördereinheit schnell, einfach und mit kostengünstigen Mitteln auf die gewünschte Weise eingestellt werden. Anhand der charakteristischen Kennlinien der Look-Up-Tabelle kann schnell erkannt und/oder ermittelt werden, bei welcher Spannung bzw. Betriebsspannung der Piezopumpe welcher Volumenstrom und welcher Gasdruck in der Abzweigungsleitung vorliegt oder vorliegen wird. Die notwendige Vorüberlegung zur Verwendung der Piezopumpe und einer zugehörigen Look-Up-Tabelle war jedoch, dass sich bei Versuchen im Rahmen der vorliegenden Erfindung herausgestellt hat, dass bereits die vorstehend erwähnten kleinen Drücke und kleinen Volumenströme zur gewünschten Ermittlung der Kohlenstoffdioxidkonzentration mittels Wärmeleitungsmessung im Messgas ausreichend sind. Für konventionelle Systeme mit Volumenströmen des Messgases von beispielsweise mehr als 100 ml/min und/oder mehr als 40 mbar wäre eine Piezopumpe weniger bis gar nicht geeignet.

Außerdem ist es möglich, dass bei einem erfindungsgemäßen Verfahren die Fluidfördereinheit, zum Erzeugen eines gleichmäßigen Volumenstroms und/oder Gasdrucks des Messgases in der Abzweigungsleitung zur Sensoreinheit, ausschließlich unter Berücksichtigung eines Atemwegsdrucks während der inspiratorischen Phase in der Hauptleitung oder ausschließlich unter Berücksichtigung eines Atemwegsdrucks während der exspiratorischen Phase der Beatmung in der Hauptleitung adaptiv eingestellt wird. Bei Versuchen im Rahmen der vorliegenden Erfindung hat sich herausgestellt, dass die Betrachtung bzw. Berücksichtigung von beispielsweise nur der inspiratorischen Phase oder der exspiratorischen Phase ausreicht, um die Kohlenstoffdioxidkonzentration mit reduzierter Rechenleistung trotzdem ausreichend genau zu ermitteln und/oder den möglichst gleichmäßigen Volumenstrom und/oder möglichst gleichmäßigen Gasdruck des Messgases zu erzeugen.

Darüber hinaus ist es möglich, dass bei einem Verfahren gemäß der vorliegenden Erfindung die Fluidfördereinheit, zum Erzeugen eines gleichmäßigen Volumenstroms und/oder Gasdrucks des Messgases in der Abzweigungsleitung zur Sensoreinheit, während einer exspiratorischen Phase der Beatmung mit gleichbleibender Leistung betrieben wird und während einer inspiratorischen Phase der Beatmung unter Berücksichtigung des Atemwegsdrucks während der inspiratorischen Phase der Beatmung adaptiv eingestellt wird. Die Fluidfördereinheit muss folglich nicht durchgehend adaptiv eingestellt und/oder betrieben werden. Auch damit kann eine erforderliche Rechenleistung zum Ermitteln der Kohlenstoffdioxidkonzentration reduziert und das Verfahren ressourcenschonend durchgeführt werden. Die Fluidfördereinheit wird hierbei während der exspiratorischen Phase der Beatmung mit gleichbleibender Leistung, insbesondere mit maximaler Leistung oder mit einer gleichbleibenden Leistung in einem Bereich zwischen 80% und 100% der maximalen Leistung der Fluidfördereinheit, betrieben.

Bei einer weiteren Ausgestaltungsvariante der vorliegenden Erfindung ist es möglich, dass bei einem Verfahren die Fluidfördereinheit, zum Erzeugen eines gleichmäßigen Volumenstroms und/oder Gasdrucks des Messgases in der Abzweigungsleitung zur Sensoreinheit, während einer exspiratorischen Phase der Beatmung unter Berücksichtigung des Atemwegsdrucks während der exspiratorischen Phase der Beatmung adaptiv eingestellt bzw. betrieben wird und während einer inspiratorischen Phase der Beatmung deaktiviert wird. Damit kann das Verfahren effizient und insbesondere energiesparend betrieben werden. Außerdem kann damit die Lebenszeit der Fluidfördereinheit verlängert werden. Darunter, dass die Fluidfördereinheit deaktiviert wird kann verstanden werden, dass die Fluidfördereinheit ausgeschaltet ist oder wird und/oder zumindest nicht betrieben wird.

Gemäß einem weiteren Aspekt der vorliegenden Erfindung wird eine Ermittlungsvorrichtung zum Ermitteln einer Kohlenstoffdioxidkonzentration in Messgas aus einem Medizingerät während einer Beatmung einer Person durch das Medizingerät nach Anspruch 8 bereitgestellt.

Damit bringt die erfindungsgemäße Ermittlungsvorrichtung die gleichen Vorteile mit sich, wie sie ausführlich mit Bezug auf das erfindungsgemäße Verfahren beschrieben worden sind. Die Ermittlungsvorrichtung kann als separate Vorrichtung zum Medizingerät oder als Bestandteil des Medizingerätes ausgestaltet sein. Die Einstelleinheit kann ein gattungsgemäßes Steuergerät des Medizingerätes umfassen oder als Bestandteil eines solchen Steuergerätes ausgestaltet sein.

Gemäß einer weiteren Ausführungsform der vorliegenden Erfindung kann eine Ermittlungsvorrichtung einen Atemwegsdruck-Sensor zum Messen des Atemwegsdrucks in der Hauptleitung aufweisen, wobei die Einstelleinheit zum adaptiven Einstellen der Fluidfördereinheit unter Berücksichtigung und/oder Verwendung des gemessenen Atemwegsdrucks in der Hauptleitung, zum Erzeugen eines gleichmäßigen Volumenstroms und/oder Gasdrucks des Messgases in der Abzweigungsleitung zur Sensoreinheit, konfiguriert und ausgestaltet ist.

Die Ermittlungsvorrichtung kann ferner zum Ermitteln der Kohlenstoffdioxidkonzentration im Messgas während einer druckgeführten Beatmung der Person durch das Medizingerät konfiguriert sein, wobei die Einstelleinheit zum adaptiven Einstellen der Fluidfördereinheit, zum Erzeugen eines gleichmäßigen Volumenstroms und/oder Gasdrucks des Messgases in der Abzweigungsleitung zur Sensoreinheit, unter Verwendung eines durch die druckgeführte Beatmung der Person eingestellten Atemwegsdrucks in der Hauptleitung, konfiguriert und ausgestaltet sein kann.

Außerdem kann die Ermittlungseinheit zum Ermitteln der Kohlenstoffdioxidkonzentration im Messgas während einer volumengeführten Beatmung der Person durch das Medizingerät konfiguriert sein, wobei die Einstelleinheit zum adaptiven Einstellen der Fluidfördereinheit, zum Erzeugen eines gleichmäßigen Volumenstroms und/oder Gasdrucks des Messgases in der Abzweigungsleitung zur Sensoreinheit, unter Verwendung eines aus der volumengeführten Beatmung der Person resultierenden Atemwegsdrucks in der Hauptleitung, konfiguriert und ausgestaltet sein kann.

Zudem weist die Fluidfördereinheit zum Fördern des Messgases aus der Hauptleitung durch die Abzweigungsleitung zur Sensoreinheit eine Piezopumpe auf, wobei die Einstelleinheit zum adaptiven Einstellen einer Betriebsspannung der Piezopumpe konfiguriert ist, um, unter Berücksichtigung des Atemwegsdrucks sowie unter Verwendung einer Look-Up-Tabelle, den gleichmäßigen Volumenstrom und/oder Gasdruck des Messgases in der Abzweigungsleitung zur Sensoreinheit zu erzeugen.

Bei einer erfindungsgemäßen Ermittlungsvorrichtung kann die Einstelleinheit ferner zum adaptiven Einstellen der Fluidfördereinheit und zum Erzeugen eines gleichmäßigen Volumenstroms und/oder Gasdrucks des Messgases in der Abzweigungsleitung zur Sensoreinheit, ausschließlich unter Berücksichtigung eines Atemwegsdrucks während einer inspiratorischen Phase der Beatmung in der Hauptleitung oder ausschließlich unter Berücksichtigung eines Atemwegsdrucks während einer exspiratorischen Phase der Beatmung in der Hauptleitung, konfiguriert und ausgestaltet sein.

Außerdem kann die Einstelleinheit einer erfindungsgemäßen Ermittlungsvorrichtung konfiguriert und ausgestaltet sein, die Fluidfördereinheit, zum Erzeugen eines gleichmäßigen Volumenstroms und/oder Gasdrucks des Messgases in der Abzweigungsleitung zur Sensoreinheit, während einer exspiratorischen Phase der Beatmung mit gleichbleibender Leistung zu betreiben und während einer inspiratorischen Phase der Beatmung unter Berücksichtigung des Atemwegsdrucks während der inspiratorischen Phase der Beatmung adaptiv einzustellen. Darüber hinaus kann die Einstelleinheit konfiguriert und ausgestaltet sein, die Fluidfördereinheit, zum Erzeugen eines gleichmäßigen Volumenstroms und/oder Gasdrucks des Messgases in der Abzweigungsleitung zur Sensoreinheit, während einer exspiratorischen Phase der Beatmung unter Berücksichtigung des Atemwegsdrucks während der exspiratorischen Phase der Beatmung adaptiv einzustellen und während einer inspiratorischen Phase der Beatmung zu deaktivieren.

Eine erfindungsgemäße Ermittlungsvorrichtung kann ferner wenigstens ein HME-Filter in und/oder an der Abzweigungsleitung aufweisen. Die Einstelleinheit ist in diesem Fall konfiguriert, um einen möglichen Einfluss des HME-Filters auf den Volumenstrom und/oder den Gasdruck in der Abzweigungsleitung dahingehend zu kompensieren, dass weiterhin ein gleichmäßiger Volumenstrom und/oder ein gleichmäßiger Gasdruck in der Abzweigungsleitung erzeugt werden. Im Rahmen der vorliegenden Erfindung wurde erkannt, dass unter Verwendung eines HME-Filters zum Filtern des Messgases, Temperatur- und Feuchtigkeitsunterschiede im Messgas, die während der inspiratorischen Phase und der exspiratorischen Phase der Person bzw. eines an dem Medizingerät angeschlossenen Patienten verursacht werden, dahingehend gepuffert, ausgeglichen, verringert und/oder geglättet werden können, dass die Kohlenstoffdioxidkonzentration im Vergleich zu einem System ohne HME-Filter deutlich genauer ermittelt bzw. gemessen und/oder berechnet werden kann. D. h., das HME-Filter trägt dazu bei, dass die Kohlenstoffdioxidkonzentration auf einfache Weise noch genauer ermittelt werden kann.

Außerdem wurde erkannt, dass das verwendete HME-Filter keinen nennenswerten und/oder nachteiligen Effekt auf andere zu messende Gaskomponenten hat. D. h., die Feuchte und die Wärme des Messgases werden zeitlich gleichmäßig verteilt, ohne den eigentlich angestrebten Messeffekt der Wärmeleitungsunterschiede hinsichtlich des vorhandenen und fehlenden Kohlenstoffdioxids zu beeinflussen. Das HME-Filter nimmt folglich keinen oder im Wesentlichen keinen Einfluss auf die Zuführung der Kohlenstoffdioxidmenge zur Sensoreinheit. Lediglich durch das Volumen des HME-Filters wird möglicherweise der Gastransport etwas verzögert. Dies nimmt jedoch keinen oder zumindest keinen nennenswerten Einfluss auf die gewünschte Ermittlung der Kohlenstoffdioxidkonzentration im Messgas. Wärmeleitungsänderungen, die aus Temperatur- und/oder Feuchtigkeitsänderungen im Messgas resultieren und synchron zu den Atemphasen auftreten, zählen neben sich veränderten Volumenströmen zu den Hauptursachen für ungenaue Kohlenstoffdioxidmessungen. Durch die Verwendung des HME-Filters kann dieser Problematik einfach, kostengünstig und effektiv Rechnung getragen werden.

Unter einem HME-Filter ist in der Medizintechnik ein Heat-Moisture-Exchange-Filter und/oder ein Filtergehäuse mit einem solchen Filtermaterial zu verstehen. Das HME-Filter kann folglich als Wärme- und Feuchtigkeitstauscher verstanden werden. HME-Filter werden bislang insbesondere in einem Mainstream bzw. in der Hauptleitung eines Beatmungsgerätes bzw. eines entsprechenden Medizingerätes eingesetzt, wo sie im Zyklus der Beatmung stets im Wechsel mit Inspirationsgas und Exspirationsgas durchströmt werden. HME-Filter dienten bislang insbesondere einer geeigneten Befeuchtung des Inspirationsgases bzw. der eingeatmeten Luft des Patienten sowie der Vermeidung von Kreuzkontaminationen in der Hauptleitung. Das vorgeschlagene HME-Filter der Ermittlungsvorrichtung ist hinsichtlich seiner Größe und/oder Funktion bevorzugt zum Puffern, Ausgleichen, Verringern und/oder Glätten von Temperatur- und/oder Feuchtigkeitsunterschieden des abgezweigten Messgases für die Dauer von mindestens eines Atemzuges, also einschließlich inspiratorischer Phase sowie exspiratorischen Phase, konfiguriert. Das HME-Filter kann demnach nicht nur zum klassischen Filtern des Messgases, sondern insbesondere zum Puffern, Ausgleichen, Verringern und/oder Glätten der Temperatur- und/oder Feuchtigkeitsunterschiede im abgezweigten Messgas bereitgestellt sein. Das wenigstens eine HME-Filter kann ein Filtergehäuse und Filtermaterial zum Filtern des Messgases im Filtergehäuse aufweisen. Das Filtergehäuse kann als starres Filtergehäuse oder als flexibles bzw. elastisch verformbares Filtergehäuse, das beispielsweise tubusförmig ausgestaltet ist, konfiguriert sein. Das HME-Filter kann ebenso ohne Filtergehäuse und ausschließlich als das funktionsrelevante HME-Filtermaterial, beispielsweise in Form eines Schlaucheinsatzes, ausgestaltet sein.

Dadurch, dass das HME-Filter bevorzugt nur mit dem abgesaugten Messgas durchströmt wird, also insbesondere nicht mit dem gesamten Gas der Hauptleitung, kann es kleiner, insbesondere um ein Vielfaches kleiner, als ein konventionelles, in der Hauptleitung verwendetes HME-Filter ausgestaltet sein. Das HME-Filter ist bevorzugt in einer Messgasströmungsrichtung zur Sensoreinheit stromaufwärts der Sensoreinheit und/oder in einem im Beatmungsgerät verbauten Zustand stromaufwärts der Sensoreinheit ausgestaltet, sodass das Messgas durch das HME-Filter hindurchströmen kann, bevor es zur Sensoreinheit gelangt.

Die Ermittlungsvorrichtung ist bevorzugt für die Verwendung in und/oder mit einem Medizingerät in Form eines Beatmungsgerätes ausgestaltet. Die Abzweigungsleitung weist vorzugsweise eine flexible Schlauchleitung zum Leiten des abgezweigten Messgases zur Sensoreinheit auf. Ferner kann die Abzweigungsleitung in Form der flexiblen Schlauchleitung ausgestaltet sein. Darüber hinaus ist es möglich, dass die Abzweigungsleitung neben der Schlauchleitung noch weitere Funktionsbauteil wie Adapter- und/oder Verbindungsbauteile zum Anschließen der Schlauchleitung an der Hauptleitung, an der Sensoreinheit und/oder am HME-Filter, aufweist.

Die Sensoreinheit kann gemäß einem in der DE 10 2010 047 159 A1 beschriebenen Sensor zum Ermitteln der Kohlenstoffdioxidkonzentration im Messgas ausgestaltet und/oder konfiguriert sein, wobei der Sensoreinheit das Messgas mittels der Fluidfördereinheit und insbesondere mittels einer Absaugpumpe zugeführt wird. Die Abzweigungsleitung weist einen kleineren, insbesondere einen um ein Vielfaches kleineren Innendurchmesser als eine gattungsgemäße Hauptleitung eines Beatmungsgerätes auf.

Gemäß einer weiteren Ausführungsform der vorliegenden Erfindung ist es möglich, dass bei einer Ermittlungsvorrichtung das wenigstens eine HME-Filter in der Abzweigungsleitung ausgestaltet ist. Damit kann die Ermittlungsvorrichtung besonders kompakt und entsprechend platzsparend zur Verfügung gestellt werden. Ferner kann die Ermittlungsvorrichtung einfach am und/oder im Medizingerät installiert werden. Das wenigstens eine HME-Filter kann bei der Installation bereits in der Abzweigungsleitung ausgestaltet sein. Das wenigstens eine HME-Filter ist insbesondere innerhalb eines Leitungsvolumens der Abzweigungsleitung ausgestaltet. Die Abzweigungsleitung kann beispielsweise eine Schlauchleitung aufweisen, wobei das wenigstens eine HME-Filter wenigstens in einem Teil des Innenvolumens der Schlauchleitung ausgestaltet ist. Mit anderen Worten, wenigstens ein Teil eines Schlauchmantels der Schlauchleitung kann das wenigstens eine HME-Filter über die gesamte Länge des wenigstens einen HME-Filters oder über einen Teil der Länge des HME-Filters mantelförmig umschließen. Das wenigstens eine HME-Filter kann sozusagen in Form eines Schlaucheinsatzes ausgestaltet sein. Das wenigstens eine HME-Filter ist bevorzugt form- und/oder kraftschlüssig in der Abzweigungsleitung ausgestaltet. Die Außenumfangsfläche des wenigstens einen HME-Filters kann demnach komplementär zu einer Innenumfangsfläche der Abzweigungsleitung, insbesondere zu einer Innenumfangsfläche der Schlauchleitung der Abzweigungsleitung, ausgestaltet sein. Der Außendurchmesser des wenigstens einen HME-Filters kann folglich dem Innendurchmesser an der Stelle der Schlauchleitung entsprechen, an welcher das wenigstens eine HME-Filter in der Schlauchleitung positioniert ist, oder zum Einsetzen des wenigstens einen HME-Filters in die Abzweigungsleitung geringfügig kleiner als der Innendurchmesser an der Stelle der Schlauchleitung sein.

Ferner ist es möglich, dass bei einer erfindungsgemäßen Ermittlungsvorrichtung die Abzweigungsleitung einen hauptleitungsseitigen Endabschnitt zum Anbinden der Abzweigungsleitung an die Hauptleitung und einen sensorseitigen Endabschnitt zum Anbinden der Abzweigungsleitung an die Sensoreinheit aufweist, wobei ein HME-Filter am und/oder im hauptleitungsseitigen Endabschnitt angeordnet ist. Das eine, insbesondere einzelne HME-Filter ist damit möglichst direkt und/oder nahe an der Hauptleitung angeordnet. Dadurch kann das bestimmungsgemäße Puffern bzw. Ausgleichen der Temperatur- und/oder Feuchtigkeitsunterschiede im Messgas durch das HME-Filter möglichst frühzeitig stromaufwärts der Sensoreinheit durchgeführt werden. Unerwünschtes Kondensat in der Abzweigungsleitung stromabwärts des HME-Filters und/oder stromaufwärts der Sensoreinheit können dadurch effektiv verhindert oder zumindest effektiv reduziert werden. Dies ist insbesondere dann von Vorteil, wenn die Abzweigungsleitung eine längere Schlauchleitung aufweist und kritische Bedingungen, beispielsweise kalte Außentemperaturen, vorherrschen, bei welchen die Temperatur in der Schlauchleitung deutlich unter die Maskentemperatur fällt bzw. den Taupunkt der mittleren Feuchte unterschreitet. Darunter, dass der sensorseitige Endabschnitt zum Anbinden der Abzweigungsleitung an die Sensoreinheit ausgestaltet ist kann verstanden werden, dass am sensorseitigen Endabschnitt ein Verbindungsanschluss zum fluiddichten Anschließen der Abzweigungsleitung an der Hauptleitung, insbesondere an einem Gegen-Verbindungsanschluss der Hauptleitung, ausgestaltet ist. Unter dem fluiddichten Anschließen kann eine Anschlussverbindung verstanden werden, durch welche das Messgas leckagefrei aus der Hauptleitung in die Abzweigungsleitung gleitet, insbesondere gesaugt, werden kann. Darunter, dass das HME-Filter am und/oder im hauptleitungsseitigen Endabschnitt angeordnet ist kann verstanden werden, dass das HME-Filter, beispielsweise in Form eines Schlaucheinsatzes, wenigstens teilweise im hauptleitungsseitigen Endabschnitt der Abzweigungsleitung bzw. einer Schlauchleitung der Abzweigungsleitung angeordnet ist, oder als Anbauteil wenigstens teilweise außerhalb einer solchen Schlauchleitung an der Schlauchleitung angeordnet ist.

Weiterhin ist es möglich, dass bei einer Ermittlungsvorrichtung gemäß der vorliegenden Erfindung die Abzweigungsleitung einen hauptleitungsseitigen Endabschnitt zum Anbinden der Abzweigungsleitung an die Hauptleitung und einen sensorseitigen Endabschnitt zum Anbinden der Abzweigungsleitung an die Sensoreinheit aufweist, wobei die Ermittlungsvorrichtung ein erstes HME-Filter am und/oder im hauptleitungsseitigen Endabschnitt und ein zweites HME-Filter am und/oder im sensorseitigen Endabschnitt aufweist. Durch das zweite HME-Filter am und/oder im sensorseitigen Endabschnitt kann die Sensoreinheit effektiv vor kondensierender Feuchtigkeit geschützt werden. Dies führt wiederum zu einer möglichst feuchtigkeitsbefreiten Messgaszufuhr zur Sensoreinheit und folglich zu entsprechend genauen Messergebnissen. Die beiden HME-Filter sind, entlang der Abzweigungsleitung betrachtet, bevorzugt beabstandet voneinander, beispielsweise um mehr als 50 cm, insbesondere in einem Bereich zwischen 50 cm und 150 cm beabstandet voneinander, ausgestaltet. Die beiden HME-Filter weisen vorzugsweise die gleiche Größe und/oder Form auf.

Außerdem ist es möglich, dass bei einer Ermittlungsvorrichtung gemäß der vorliegenden Erfindung das erste HME-Filter im hauptleitungsseitigen Endabschnitt der Abzweigungsleitung in Form eines Schlaucheinsatzes ausgestaltet ist, wobei die Abzweigungsleitung, in einer Strömungsrichtung des Messgases durch die Abzweigungsleitung betrachtet, auf Höhe des HME-Filters einen größeren Innendurchmesser als in einem Bereich stromabwärts des HME-Filters aufweist. Dadurch, dass die Abzweigungsleitung stromabwärts des HME-Filters weniger anfällig gegenüber kondensierender Feuchte im Messgas ist, kann der Innendurchmesser der Abzweigungsleitung stromabwärts des HME-Filters relativ klein ausgestaltet sein. Damit können Material und Kosten gespart werden und die Abzweigungsleitung kann kompakt ausgestaltet sein. Insbesondere können dadurch ein Totraum in der Abzweigungsleitung und/oder eine Messverzögerung relativ gering gehalten werden. Die Strömungsrichtung des Messgases durch die Abzweigungsleitung ist in einem Zustand der Ermittlungsvorrichtung, in welchem diese in der Medizineinheit verbaut ist, zu betrachten. So verläuft die Strömungsrichtung von der Hauptleitung durch die Abzweigungsleitung, dort durch das in und/oder an der Abzweigungsleitung angeordnete wenigstens eine HME-Filter, und stromabwärts des wenigstens einen HME-Filters hin zur Sensoreinheit und darüber hinaus, beispielsweise zu einer Pumpe, die stromabwärts der Sensoreinheit zum Absaugen des Messgases aus der Hauptleitung in die Abzweigungsleitung angeordnet sein kann. Der Innendurchmesser auf Höhe des HME-Filters ist im Vergleich zum Innendurchmesser stromabwärts des HME-Filters etwas größer ausgestaltet, um das HME-Filter mit einem entsprechend großen Durchmesser bzw. Außendurchmesser aufnehmen zu können. Damit können den Wünschen nach einer ausreichenden Pufferwirkung durch das HME-Filter und trotzdem einer platzsparenden und möglichst verzögerungsarmen Weiterleitung des Messgases zur Sensoreinheit Rechnung getragen werden.

Der Innendurchmesser der Abzweigungsleitung kann bei einer erfindungsgemäßen Ermittlungsvorrichtung auf Höhe des ersten HME-Filters einen Wert in einem Bereich zwischen 2 mm und 4 mm aufweisen und der Innendurchmesser der Abzweigungsleitung stromabwärts des ersten HME-Filters kann einen Wert in einem Bereich zwischen 0,5 mm und 2 mm aufweisen. Bei umfangreichen Versuchen im Rahmen der vorliegenden Erfindung hat sich herausgestellt, dass mit einem Durchmesser im Bereich zwischen 2 mm und 4 mm, mögliches Kondensat stromaufwärts des HME-Filters relativ unproblematisch ist. Der Durchmesser in einem Bereich zwischen 0,5 mm und 2 mm stromabwärts des HME-Filters hat sich als vorteilhafter Kompromiss hinsichtlich einer robusten Abzweigungsleitung und trotzdem einem möglichst geringen Totraum bzw. einer entsprechend geringen Messverzögerung herausgestellt. Die Abzweigungsleitung bzw. Schlauchleitung kann zum Herstellen einer Strömungsgeschwindigkeit in einem Bereich zwischen 1 m/s und 1,5 m/s bei einem Volumenstrom in einem Bereich zwischen 50 ml/min und 70 ml/min ausgestaltet sein.

Das wenigstens eine HME-Filter kann bei einer Ermittlungsvorrichtung gemäß der vorliegenden Erfindung im hauptleitungsseitigen Endabschnitt der Abzweigungsleitung ferner in Form eines Schlaucheinsatzes ausgestaltet sein, wobei die Abzweigungsleitung, in Strömungsrichtung des Messgases durch die Abzweigungsleitung betrachtet, in einem Bereich stromaufwärts des wenigstens einen HME-Filters einen größeren Innendurchmesser als stromabwärts des wenigstens einen HME-Filters aufweist. Damit kann verhindert werden, dass Kondensat stromaufwärts des wenigstens einen HME-Filters zu einer Verstopfung der Abzweigungsleitung führt und stromabwärts des wenigstens einen HME-Filters der gewünschte Kompromiss hinsichtlich einer robusten Abzweigungsleitung und trotzdem einem möglichst geringen Totraum bzw. einer entsprechend geringen Messverzögerung geschaffen werden kann. Als vorteilhaft hat es sich herausgestellt, wenn der Innendurchmesser der Abzweigungsleitung stromaufwärts des wenigstens einen HME-Filters einen Wert in einem Bereich zwischen 1,5 mm und 4 mm aufweist und der Innendurchmesser der Abzweigungsleitung stromabwärts des wenigstens einen HME-Filters einen Wert in einem Bereich zwischen 0,5 mm und 2 mm aufweist.

Vorteile hinsichtlich einer einfachen Fertigung der Abzweigungsleitung können erzielt werden, wenn die Bereiche stromaufwärts des HME-Filters sowie auf der Höhe des HME-Filters den gleichen Innendurchmesser aufweisen. So kann beispielsweise eine Schlauchleitung der Abzweigungsleitung mit einem Innendurchmesser, der von einem Bereich stromaufwärts des HME-Filters im sensorseitigen Endabschnitt bis hin zu einem Bereich, in welchem das HME-Filter in der Schlauchleitung ausgestaltet ist, den gleichen Wert aufweist, und erst stromabwärts des HME-Filters einen kleineren Innendurchmesser als stromaufwärts des HME-Filter oder im Bereich des HME-Filters aufweist, konfiguriert sein. Das Gleiche kann auf analoge Weise hinsichtlich eines Außendurchmessers einer solchen Schlauchleitung konfiguriert sein. Außerdem ist es möglich, dass der Beriech stromaufwärts des HME-Filters bzw. das entsprechende Innenvolumen einer Schlauchleitung der Abzweigungsleitung einen kleineren Innendurchmesser als im Bereich des HME-Filters, und bevorzugt trotzdem größer als im Bereich stromabwärts des HME-Filters, aufweist. Der Innendurchmesser einer vorstehend beschriebenen Schlauchleitung kann über den Bereich stromaufwärts des HME-Filters zu dem Bereich, in welchem das HME-Filter in der Schlauchleitung ausgestaltet ist, mithin konstant bleiben und sich von dem Bereich, in welchem das HME-Filter in der Schlauchleitung ausgestaltet ist, hin zu dem Bereich stromabwärts des HME-Filters, verkleinern, oder von dem Bereich stromaufwärts des HME-Filters hin zu dem Bereich, in welchem das HME-Filter in der Schlauchleitung ausgestaltet ist, vergrößern, und von dem Bereich, in welchem das HME-Filter in der Schlauchleitung ausgestaltet ist, hin zu dem Bereich stromabwärts des HME-Filters, wieder verkleinern.

Bei einer Ermittlungsvorrichtung gemäß der vorliegenden Erfindung kann das wenigstens eine HME-Filter ferner eine Länge in einem Bereich zwischen 8 mm und 20 mm und eine Breite in einem Bereich zwischen 2 mm und 6 mm aufweisen. Insbesondere weist das wenigstens eine HME-Filter eine Länge in einem Bereich zwischen 10 mm und 15 mm und eine Breite in einem Bereich zwischen 3 mm und 5 mm auf. Das wenigstens eine HME-Filter wird erfindungsgemäß möglichst nur mit dem Messgas bzw. dem Absaugestrom durchströmt und kann somit relativ klein gehalten werden. Die bekannten und bislang in der Hauptleitung verwendeten HME-Filter sind für Patientengasströme von bis zu 180 l/min ausgelegt. Das wenigstens eine HME-Filter gemäß der vorliegenden Erfindung ist für eine Durchströmung mit Messgas in einem Bereich von beispielsweise zwischen 30 ml/min und 100 ml/min, insbesondere in einem Bereich zwischen 40 ml/min und 70 ml/min, ausgelegt. Die Abzweigungsleitung kann deshalb entsprechend klein, material- und platzsparend sowie kostengünstig ausgestaltet sein. Das wenigstens eine HME-Filter ist bevorzugt zylinderförmig und mit einer Länge in einem Bereich zwischen 8 mm und 20 mm und einem Durchmesser in einem Bereich zwischen 2 mm und 6 mm ausgestaltet.

Gemäß einer weiteren Ausgestaltungsvariante der vorliegenden Erfindung ist es möglich, dass bei einer Ermittlungsvorrichtung die Abzweigungsleitung eine Schlauchleitung mit einer Länge in einem Bereich zwischen 80 cm und 150 cm aufweist. Bei Versuchen im Rahmen der vorliegenden Erfindung wurde herausgefunden, dass bereits bei dieser Schlauchlänge ein wirksamer Puffereffekt hinsichtlich des gewünschten Temperatur- und/oder Feuchtigkeitsausgleichs erzielt werden kann. Die Schlauchleitung weist insbesondere eine Länge in einem Bereich zwischen 90 cm und 110 cm auf. Die Schlauchleitung weist den vorstehend beschriebenen Innendurchmesser in einem Bereich zwischen 0,5 mm und 2 mm, vorzugsweise über eine Länge der Schlauchleitung in einem Bereich zwischen 80 cm und 120 cm, auf.

Weiterhin kann die Abzweigungsleitung bei einer erfindungsgemäßen Ermittlungsvorrichtung eine Schlauchleitung aus Silikon oder zumindest teilweise aus Silikon aufweisen. Bei Versuchen im Rahmen der vorliegenden Erfindung hat sich gezeigt, dass unter Verwendung eines Silikonschlauches in der Abzweigungsleitung ein Gegentrocknungseffekt auf das Messgas ausgeübt wird, der zu einer weiteren Pufferung und/oder Glättung von Feuchtigkeitsschwankungen führt.

Von weiterem Vorteil kann es bei einer Ermittlungsvorrichtung gemäß der vorliegenden Erfindung sein, wenn die Abzweigungsleitung eine Schlauchleitung mit einer PVC-Beschichtung an einer Außenumfangsfläche der Schlauchleitung aufweist. Durch die PVC-Beschichtung können Umwelteinflüsse auf das Messgas, die zu einer Beeinflussung des Messergebnisses führen könnten, auf einfache und kostengünstige Weise verhindert werden. Die PVC-Beschichtung weist bevorzugt eine Dicke in einem Bereich zwischen 0,1 mm und 0,4 mm auf.

Bei einer Ermittlungsvorrichtung gemäß einer weiteren Ausgestaltungsvariante der vorliegenden Erfindung ist es möglich, dass die Abzweigungsleitung einen Luer-Lock-Anschluss zum Herstellen einer Fluidverbindung mit der Hauptleitung aufweist. Damit lässt sich die Abzweigungsleitung besonders schnell und einfach mit der Hauptleitung und/oder einem Anschlussabschnitt der Hauptleitung verbinden bzw. an dieser anschließen. An der Hauptleitung, der Atemmaske und/oder einem Exspirationsventil an der Atemmaske des Medizinsystems kann entsprechend ein Gegen-Luer-Lock-Anschluss für eine entsprechende Anschlussverbindung zwischen der Hauptleitung und der Abzweigungsleitung, zwischen der Atemmaske und der Abzweigungsleitung und/oder zwischen dem Exspirationsventil und der Abzweigungsleitung ausgestaltet sein.

Das wenigstens eine HME-Filter kann bei einer bevorzugten Ausführungsform einer erfindungsgemäßen Ermittlungsvorrichtung ferner einen mikroporösen Kunststoffschaum aufweisen. Damit lassen sich die gewünschten Ausgleichseffekte auf die Temperatur und/oder die Feuchtigkeit im Messgas besonders zuverlässig erreichen. Das wenigstens eine HME-Filter kann insbesondere einen offenporigen, salzbeschichteten Kunststoffschaum mit aufweisen. Das wenigstens eine HME-Filter kann deshalb mit Bezug auf das Inspirationsgas eine Befeuchtungseffizienz von ca. 30 mg Wasser pro Liter aufweisen.

Bei einer erfindungsgemäßen Ermittlungsvorrichtung kann die Fluidfördereinheit ferner eine Piezopumpe aufweisen. Wie vorstehend bereits erwähnt, kann unter Verwendung der Piezopumpe eine zugehörige Look-Up-Tabelle für ein schnelles, einfaches und trotzdem genaues Ermitteln der Kohlenstoffdioxidkonzentration und/oder für ein schnelles und zuverlässiges Erzeugen eines möglichst gleichmäßigen Volumenstroms und/oder eines möglichst gleichmäßigen Gasdrucks des Messgases verwendet werden. Eine erfindungsgemäße Ermittlungsvorrichtung kann außerdem zum Ermitteln einer Kohlenstoffdioxidkonzentration in Messgas aus einem Medizingerät während einer druckgeführten Beatmung einer Person durch das Medizingerät konfiguriert sein, wobei die Einstelleinheit zum adaptiven Einstellen der Fluidfördereinheit unter Verwendung eines durch die druckgeführte Beatmung der Person eingestellten Atemwegsdrucks in der Hauptleitung konfiguriert ist. Darunter, dass die Einstelleinheit zum adaptiven Einstellen der Fluidfördereinheit unter Verwendung eines durch die druckgeführte Beatmung der Person eingestellten Atemwegsdrucks in der Hauptleitung konfiguriert ist kann verstanden werden, dass die Einstelleinheit den eingestellten Atemwegsdruck in der Hauptleitung zum adaptiven Einstellen der Fluidfördereinheit verwendet bzw. basierend auf dem eingestellten Atemwegsdruck die Fluidfördereinheit adaptiv einstellt.

Bei einer weiteren Ausgestaltungsvariante der vorliegenden Erfindung kann die Ermittlungsvorrichtung zum Ermitteln einer Kohlenstoffdioxidkonzentration in Messgas aus einem Medizingerät während einer volumengeführten Beatmung einer Person durch das Medizingerät konfiguriert sein, wobei die Einstelleinheit zum adaptiven Einstellen der Fluidfördereinheit unter Verwendung eines aus der volumengeführten Beatmung der Person resultierenden Atemwegsdrucks in der Hauptleitung konfiguriert ist. Darunter, dass die Einstelleinheit zum adaptiven Einstellen der Fluidfördereinheit unter Verwendung eines aus der volumengeführten Beatmung der Person resultierenden Atemwegsdrucks in der Hauptleitung konfiguriert ist kann verstanden werden, dass die Einstelleinheit den aus der volumengeführten Beatmung der Person resultierenden Atemwegsdrucks in der Hauptleitung zum adaptiven Einstellen der Fluidfördereinheit verwendet bzw. die Fluidfördereinheit basierend auf dem resultierenden Atemwegsdrucks adaptiv einstellt.

Gemäß einem weiteren Aspekt der vorliegenden Erfindung wird ein Medizingerät umfassend eine Ermittlungsvorrichtung nach Anspruch 8 zum Beatmen einer Person zur Verfügung gestellt.

Bei einem erfindungsgemäßen Medizingerät kann die Hauptleitung einen Inspirationsgasleitungsabschnitt zum Leiten des Inspirationsgases und einen Gesamtgasleitungsabschnitt zum Leiten des Inspirationsgases sowie des Exspirationsgases aufweisen, wobei die Abzweigungsleitung zum Abzweigen des Messgases aus dem Gesamtgasleitungsabschnitt ausgestaltet ist. D. h., das Messgas kann aus einem Teil der Hauptleitung abgezweigt werden, durch welchen während des Betriebs des Medizingeräts sowohl Inspirationsgas als auch Exspirationsgas geleitet wird. Die Kohlenstoffdioxidkonzentration im Messgas wird insbesondere über eine Kohlenstoffdioxiddifferenz zwischen dem Inspirationsgas und dem Exspirationsgas ermittelt bzw. gemessen und mittels einer Recheneinheit des Medizingerätes berechnet.

Bei einem erfindungsgemäßen Medizingerät kann sich das wenigstens eine HME-Filter innerhalb des Gesamtgasleitungsabschnitts befinden. D.h., die Abzweigungsleitung ist nicht nur an der Hauptleitung angeschlossen und/oder angebunden, sondern erstreckt sich in die Hauptleitung, genauer gesagt in den Gesamtgasleitungsabschnitt, hinein. Das HME-Filter und/oder die Abzweigungsleitung mit einem darin angeordneten HME-Filter kann sozusagen innerhalb der Hauptleitung angeordnet und/oder geführt sein. Die Außenumfangsfläche der Abzweigungsleitung kann in einem Bereich, in welchem das HME-Filter in und/oder an der Abzweigungsleitung ausgestaltet ist, von einer Innenumfangsfläche der Hauptleitung beabstandet sein. Dadurch kann eine besonders kompakte und trotzdem funktionale Bauweise erzielt werden. Die Hauptleitung kann sich ferner zu einem Exspirationsventil des Medizingerätes hin oder durch wenigstens einen Teil des Exspirationsventils hindurch erstrecken. In diesem Fall kann das wenigstens eine HME-Filter auch als innerhalb des Exspirationsfilters ausgestaltet betrachtet werden. Auch dies führt zu einer besonders kompakten und robusten Bauweise. Insbesondere kann das HME-Filter innerhalb der Hauptleitung und/oder des Exspirationsventils effektiv vor Umwelteinflüssen geschützt werden. Bei einem Medizingerät gemäß der vorliegenden Erfindung kann sich wenigstens ein Teil der Abzweigungsleitung von einer Position innerhalb der Hauptleitung aus dem Gesamtgasleitungsabschnitt in den Inspirationsgasleitungsabschnitt erstreckten D.h., die Abzweigungsleitung kann innerhalb der Hauptleitung bzw. durch ein Hauptleitungsvolumen der Hauptleitung, die zum Führen des Inspirationsgases ausgestaltet ist, geführt sein. Mit anderen Worten, die Abzweigungsleitung kann zumindest in einen Teil der Hauptleitung integriert und/oder in dieser geführt sein. Damit kann das Medizingerät besonders platzsparend bereitgestellt werden.

Bei einem erfindungsgemäßen Medizingerät, bei welchem im Gesamtgasleitungsabschnitt ein Exspirationsventil zum Auslassen von Exspirationsgas aus dem Medizingerät in die Umgebung des Medizingerätes ausgestaltet ist, kann das wenigstens eine HME-Filter in dem Exspirationsventil ausgestaltet sein. Auch eine solche Ausgestaltungsvariante lässt sich relativ kompakt realisieren. Mit einem in das Exspirationsventil integrierten HME-Filter muss bei einem Zusammenbau des Medizingerätes lediglich die Abzweigungsleitung am Exspirationsventil angeschlossen werden und anschließend zur Sensoreinheit geführt werden. Die Abzweigungsleitung kann, beispielsweise in Form einer einfachen Schlauchleitung, im Bedarfsfall schnell, einfach und kostengünstig ersetzt werden. Unter einer Position innerhalb des Exspirationsventils bedeutet, dass das wenigstens eine HME-Filter und/oder ein Teil der Abzweigungsleitung mit dem daran und/oder darin angeordneten wenigstens einen HME-Filter in einem Ventilvolumen des Exspirationsventils angeordnet sind, durch welches das Exspirationsgas sowie das Inspirationsgas der Hauptleitung strömen. Die Abzweigungsleitung ist zum Abzweigen des Messgases aus der Hauptleitung bevorzugt am Exspirationsventil angeschlossen. Dazu kann die Abzweigungsleitung einen Abzweigungsanschluss aufweisen und das Exspirationsventil kann einen Gegen-Abzweigungsanschluss, zum Herstellen einer fluiddichten Verbindung mit dem Abzweigungsanschluss, aufweisen.

Das vorliegend beschriebene Medizingerät ist bevorzugt in Form eines Beatmungsgeräts zur Verfügung gestellt und/oder ausgestaltet. Unter dem Medizingerät kann mithin ein medizinisches Gerät zum Beatmen einer Person, insbesondere eines Patienten, verstanden werden. Das Medizingerät kann hierzu auch in Form eines Anästhesiegerätes konfiguriert sein. Das Beatmungsgerät kann vorzugweise in Form eines Notfall-Beatmungsgerätes, eines Beatmungsgerätes für die Verwendung auf einer Intensivstation, eines Heim-Beatmungsgerätes, eines mobilen Beatmungsgerätes und/oder eines Neonatal-Beatmungsgerätes konfiguriert und/oder ausgestaltet sein.

Darüber hinaus wird im Rahmen der vorliegenden Erfindung ein Computerprogrammprodukt vorgeschlagen, das Befehle umfasst, die bei der Ausführung des Computerprogrammprodukts durch einen Computer diesen veranlassen, das vorstehend beschriebene Verfahren auszuführen. Das Computerprogrammprodukt kann als computerlesbarer Anweisungscode in jeder geeigneten Programmiersprache wie beispielsweise in JAVA, C++, C# und/oder Python implementiert sein. Das Computerprogrammprodukt kann auf einem computerlesbaren Speichermedium wie einer Datendisk, einem Wechsellaufwerk, einem flüchtigen oder nichtflüchtigen Speicher, oder einem eingebauten Speicher/Prozessor abgespeichert sein. Der Anweisungscode kann einen Computer oder andere programmierbare Geräte wie ein Steuergerät und/oder die Einstelleinheit derart programmieren, dass die gewünschten Funktionen ausgeführt werden. Ferner kann das Computerprogrammprodukt in einem Netzwerk wie beispielsweise dem Internet bereitgestellt werden und/oder sein, von dem es bei Bedarf von einem Nutzer heruntergeladen werden kann. Das Computerprogrammprodukt kann sowohl mittels einer Software, als auch mittels einer oder mehrerer spezieller elektronischer Schaltungen, d.h. in Hardware oder in beliebig hybrider Form, d.h. mittels Software-Komponenten und Hardware-Komponenten, realisiert werden und/oder sein. Ein weiterer Aspekt der vorliegenden Erfindung betrifft ein Speichermittel, auf dem ein solches Computerprogrammprodukt gespeichert ist.

### BEVORZUGTE AUSFÜHRUNGSBEISPIELE

Weitere, die Erfindung verbessernde Maßnahmen ergeben sich aus der nachfolgenden Beschreibung zu verschiedenen Ausführungsbeispielen der Erfindung, welche in den Figuren schematisch dargestellt sind. Sämtliche aus den Ansprüchen, der Beschreibung oder den Figuren hervorgehende Merkmale und/oder Vorteile, einschließlich konstruktiver Einzelheiten und räumlicher Anordnungen können sowohl für sich als auch in den verschiedenen Kombinationen erfindungswesentlich sein. Es zeigen jeweils schematisch:
- Figur 1: ein Medizingerät mit einer Ermittlungsvorrichtung gemäß einer ersten Ausführungsform der vorliegenden Erfindung,
- Figur 2: ein Speichermittel mit einem darauf gespeicherten Computerprogrammprodukt,
- Figur 3: ein Medizingerät mit einer Ermittlungsvorrichtung gemäß einer zweiten Ausführungsform der vorliegenden Erfindung,
- Figur 4: ein Flussdiagramm zum Erläutern eines Verfahrens gemäß einer bevorzugten Ausführungsform der vorliegenden Erfindung,
- Figur 5: Kennliniendiagramme zum Darstellen eines Atemwegsdrucks bei einer Beatmung mit gleichmäßigem Volumenstrom durch eine erfindungsgemäße Ansteuerung einer Fluidfördereinheit,
- Figur 6: eine Look-Up-Tabelle für eine Piezopumpe,
- Figur 7: Kennliniendiagramme zum Erläutern eines adaptiven Einstellens einer Fluidfördereinheit während einer druckgeführten Beatmung durch ein erfindungsgemäßes Medizingerät, und
- Figur 8: Kennliniendiagramme zum Erläutern eines adaptiven Einstellens einer Fluidfördereinheit während einer volumengeführten Beatmung durch ein erfindungsgemäßes Medizingerät.

Elemente mit gleicher Funktion und Wirkungsweise sind in den Figuren jeweils mit den gleichen Bezugszeichen versehen.

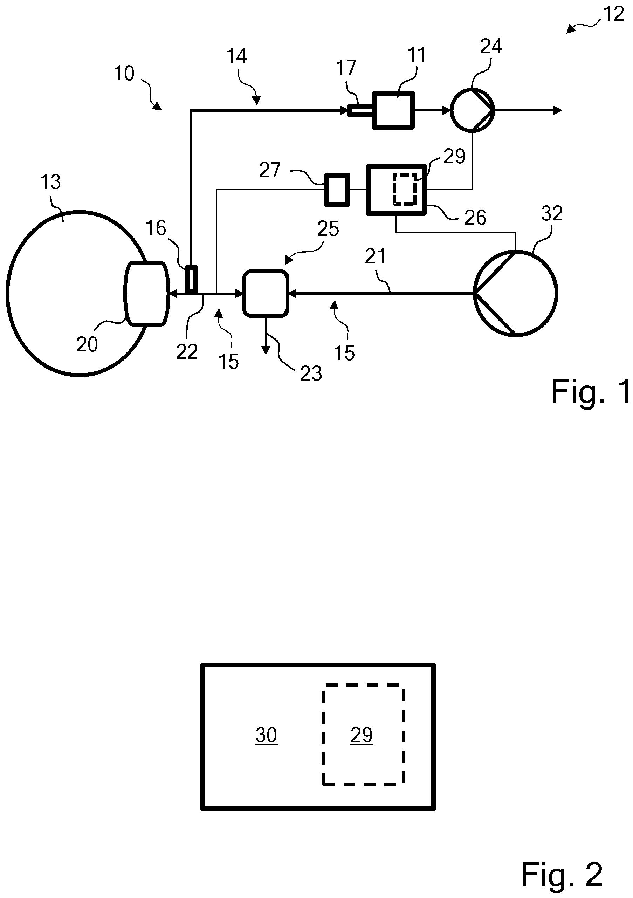

Fig. 1 zeigt ein Medizingerät 12 in Form eines Beatmungsgerätes zum Beatmen einer Person 13 gemäß einer ersten Ausführungsform. Das Medizingerät 12 umfasst eine Atemmaske 20 und eine Hauptleitung 15 zum Leiten von Inspirationsgas zur Atemmaske 20 hin und zum Leiten von Exspirationsgas von der Atemmaske 20 weg. Die Hauptleitung 15 weist einen Inspirationsgasleitungsabschnitt 21 und einen Exspirationsgasleitungsabschnitt 23 auf. Im Inspirationsgasleitungsabschnitt 21 ist eine Hauptpumpe 32 zum Zuführen des Inspirationsgases zur Atemmaske 20 bzw. zur Person 13 ausgestaltet. Stromabwärts der Hauptpumpe 32, betrachtet in einer Strömungsrichtung des Inspirationsgases, ist ein Exspirationsventil 25 ausgestaltet. Stromaufwärts des Exspirationsventils 25 und stromabwärts der Hauptpumpe 32 wird im Inspirationsgasleitungsabschnitt 21 ausschließlich Inspirationsgas geführt. Im Exspirationsventil 25, durch welches sich auch die Hauptleitung 15 erstreckt, werden Inspirationsgas zur Atemmaske 20 hin und Exspirationsgas von der Atemmaske 20 weg und über das Exspirationsventil 25 in die Umgebung des Medizingerätes 12 geführt. Zwischen dem Exspirationsventil 25 und der Atemmaske 20 ist ein Gesamtgasleitungsabschnitt 22 ausgestaltet, in welchem während der inspiratorischen Phase Inspirationsgas und während der exspiratorischen Phase Exspirationsgas geführt werden.

Das gezeigte Medizingerät 12 weist ferner eine Ermittlungsvorrichtung 10 zum Ermitteln einer Kohlenstoffdioxidkonzentration in Messgas aus dem Medizingerät 12 bzw. aus der Hauptleitung 15 während einer Beatmung der Person 13 durch das Medizingerät 12 auf. Die Ermittlungsvorrichtung 10 weist eine Abzweigungsleitung 14 mit einem ersten HME-Filter 16 und einem zweiten HME-Filter 17 zum Filtern des abgezweigten Messgases auf. Die Ermittlungsvorrichtung 10 weist ferner eine Sensoreinheit 11 zum Ermitteln einer Kohlenstoffdioxidkonzentration im Messgas auf. Die Abzweigungsleitung 14 ist zum Abzweigen des Messgases aus der Hauptleitung 15 des Medizingerätes 12 während einer inspiratorischen Phase der Person 13 als Inspirationsgas und während einer exspiratorischen Phase der Person 13 als Exspirationsgas zur Sensoreinheit 11 konfiguriert. Das erste HME-Filter 16 ist mit Blick auf eine Strömungsrichtung des abgezweigten und abgesaugten Messgases stromaufwärts der Sensoreinheit 11 direkt am Gesamtgasleitungsabschnitt 22 angeordnet und das zweite HME-Filter 17 ist mit Blick auf eine Strömungsrichtung des abgezweigten und abgesaugten Messgases stromaufwärts der Sensoreinheit 11 direkt an der Sensoreinheit 11 angeordnet. Die beiden HME-Filter 16, 17 sind jeweils zylinderförmig ausgestaltet und weisen jeweils eine Länge von 13 mm und einen Durchmesser von 3 mm auf.

Zum Absaugen des Messgases aus der Hauptleitung 15 bzw. aus dem Gesamtgasleitungsabschnitt 22 weist die Ermittlungsvorrichtung 10 eine Fluidfördereinheit 24 in Form einer Piezopumpe auf. Die Fluidfördereinheit 24 ist stromabwärts der Sensoreinheit 11 angeordnet. Die gezeigten HME-Filter 16, 17 weisen jeweils einen mikroporösen Kunststoffschaum zum Filtern des Messgases, bzw. zum Erzielen der gewünschten Puffer- bzw. Ausgleichsfunktion hinsichtlich der auftretenden Temperatur- und Feuchtigkeitsunterschiede im Messgas, auf.

Zum Ermitteln einer Kohlenstoffdioxidkonzentration im Messgas wird in der Sensoreinheit 11 insbesondere die Wärmeleitfähigkeit des Exspirationsgases gemessen. Die Messung erfolgt durch ein mikrostrukturiertes Heizelement auf einer dünnen Membran der Sensoreinheit. Neben dem Heizelement befindet sich eine thermophile Anordnung, die eine Übertemperatur des Gases nahe dem Heizelement in Bezug auf einen Siliziumrahmen der Membran misst. Weitere Details hierzu können der deutschen Patentanmeldung DE 10 2010 047 159 A1 entnommen werden.

Das Medizingerät 12 bzw. die Ermittlungsvorrichtung 10 weist ferner eine Einstelleinheit 26 auf, die zum adaptiven Einstellen der Fluidfördereinheit 24 unter Berücksichtigung eines Atemwegsdrucks in der Hauptleitung 15, zum Erzeugen eines während der inspiratorischen Phase und der exspiratorischen Phase möglichst gleichmäßigen Volumenstroms und möglichst gleichmäßigen Gasdrucks des Messgases in der Abzweigungsleitung 14 zur Sensoreinheit 11, konfiguriert und ausgestaltet ist. Die Einstelleinheit 26 kann als Steuergerät des Medizingerätes 12 betrachtet werden. Die Einstelleinheit 26 steht mit der Hauptpumpe 32 und mit der Fluidfördereinheit 24 zum Einstellen bzw. Ansteuern derselben in Signalverbindung. Die Ermittlungsvorrichtung 10 weist außerdem einen Atemwegsdruck-Sensor 27 zum Messen des Atemwegsdrucks in der Hauptleitung 15 auf, wobei die Einstelleinheit 26 zum adaptiven Einstellen der Fluidfördereinheit 24 unter Berücksichtigung des durch den Atemwegsdruck-Sensor 27 gemessenen Atemwegsdrucks in der Hauptleitung 15, zum Erzeugen des möglichst gleichmäßigen Volumenstroms sowie des möglichst gleichmäßigen Gasdrucks des Messgases in der Abzweigungsleitung 14 zur Sensoreinheit 11, konfiguriert und ausgestaltet ist.

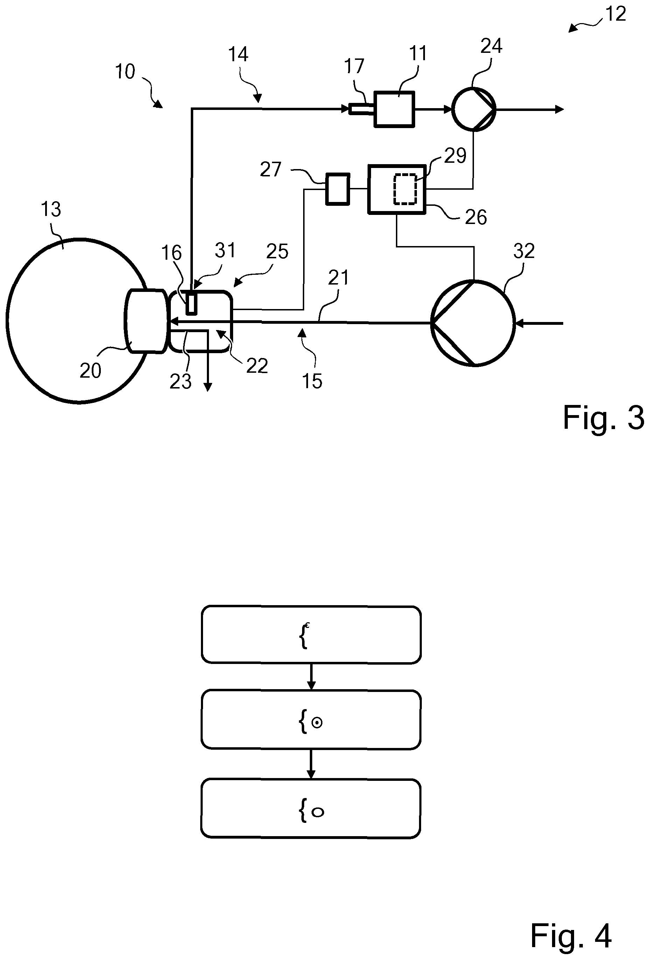

In der Einstelleinheit 26 ist ein Computerprogrammprodukt 29 installiert, das Befehle umfasst, die bei der Ausführung des Computerprogrammprodukts 29 durch die Einstelleinheit 26 diese veranlassen, das mit Bezug auf Fig. 4 beschriebene Verfahren auszuführen. In Fig. 2 ist ein Speichermittel 30 gezeigt, auf dem ein solches Computerprogrammprodukt 29 gespeichert ist.

Fig. 3 zeigt ein Medizingerät 12 gemäß einer zweiten Ausführungsform. Bei dem in Fig. 3 gezeigten Medizingerät 12 ist das Exspirationsventil 25 an der Atemschutzmaske 20 befestigt. Das in Fig. 3 gezeigte Exspirationsventil 25 weist ferner ein erstes HME-Filter 16 auf, das in das Exspirationsventil 25 integriert ist. Das erste HME-Filter 16 ist gemäß Fig. 3 direkt an einer Schlauchleitung der Abzweigungsleitung 14 ausgestaltet. Die Abzweigungsleitung 14 ist mittels der Schlauchleitung somit am Exspirationsventil 25 angeschlossen und bildet dort eine Fluidverbindung zum ersten HME-Filter 16 bzw. ermöglicht eine Fluidverbindung aus der Hauptleitung 15 durch das erste HME-Filter 16 zur Sensoreinheit 11. Hierfür weist das Exspirationsventil 25 einen Ventilanschluss 31 in Form eines Luer-Lock-Anschlusses zum Anschließen der Abzweigungsleitung 14 bzw. der Schlauchleitung auf.

Fig. 4 zeigt ein Flussdiagramm zum Erläutern eines Verfahrens zum Ermitteln einer Kohlenstoffdioxidkonzentration in Messgas. In einem ersten Schritt S1 wird das Messgas zunächst aus der Hauptleitung 15 eines wie vorstehend beschriebenen Medizingerätes 12 während einer inspiratorischen Phase einer am Medizingerät 12 angeschlossenen Person 13 als Inspirationsgas und während einer exspiratorischen Phase der Person 13 als Exspirationsgas zur Sensoreinheit 11 durch die Abzweigungsleitung 14 abgezweigt. Hierbei wird das Messgases mittels der Fluidfördereinheit 24 aus der Hauptleitung 15 durch die Abzweigungsleitung 14 zur Sensoreinheit 11 gefördert bzw. abgesaugt. In einem zweiten Schritt S2, der teilweise gleichzeitig zum ersten Schritt S1 durchgeführt wird, wird die Fluidfördereinheit 24 unter Berücksichtigung des Atemwegsdrucks in der Hauptleitung 15, zum Erzeugen eines während der inspiratorischen Phase und der exspiratorischen Phase möglichst gleichmäßigen Volumenstroms sowie eines möglichst gleichmäßigen Gasdrucks des Messgases in der Abzweigungsleitung 14 zur Sensoreinheit 11, adaptiv eingestellt. Der Atemwegsdruck in der Hauptleitung 15 wird dabei durch einen Atemwegsdruck-Sensor 27 gemessen und die Fluidfördereinheit 24 wird, zum Erzeugen des gleichmäßigen Volumenstroms und des gleichmäßigen Gasdrucks des Messgases in der Abzweigungsleitung 14 zur Sensoreinheit 11, unter Verwendung des gemessenen Atemwegsdrucks in der Hauptleitung 15 adaptiv eingestellt. Währenddessen wird in einem dritten Schritt S3 die Kohlenstoffdioxidkonzentration im Messgas mittels der Sensoreinheit 11 ermittelt. Genauer gesagt wird durch die Sensoreinheit 11 die Wärmeleitfähigkeit des Messgases gemessen um basierend darauf wie vorstehend beschrieben die Kohlenstoffdioxidkonzentration zu ermitteln.

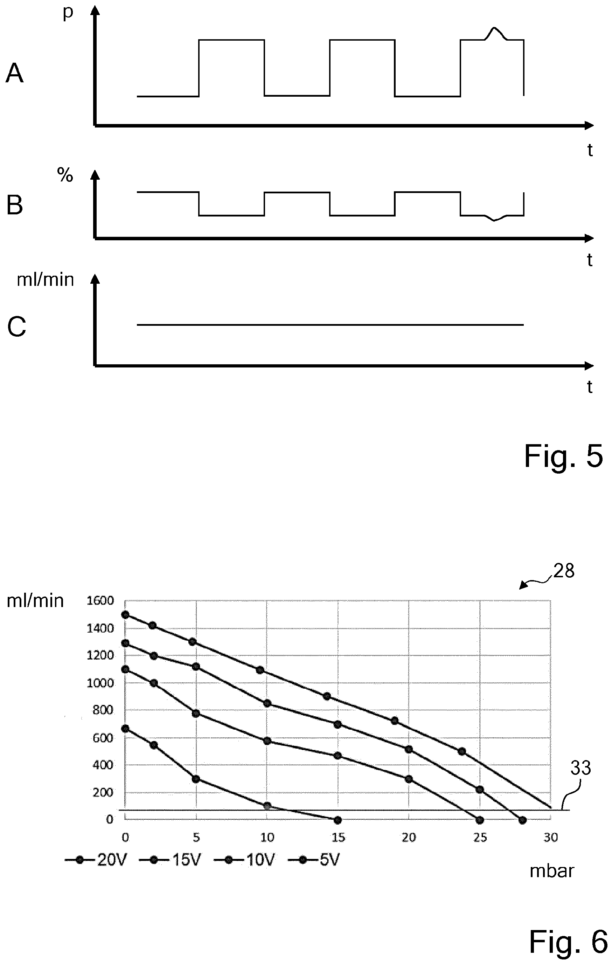

Fig. 5 zeigt Kennliniendiagramme zum Darstellen eines Atemwegsdrucks bei einer Beatmung mit gleichmäßigem Volumenstrom durch eine wie vorstehend beschriebene Ansteuerung der Fluidfördereinheit 24. Genauer gesagt zeigt Diagramm A einen typischen Verlauf des Atemwegsdrucks. Diagramm B zeigt, wie die Fluidfördereinheit 24 angesteuert bzw. eingestellt wird, um den in Diagramm C gezeigten, möglichst gleichmäßigen Volumenstrom zu erzeugen.

Fig. 6 zeigt eine Look-Up-Tabelle 28, unter Verwendung von welcher der möglichst gleichmäßige Volumenstrom sowie der möglichst gleichmäßige Gasdruck des Messgases in der Abzweigungsleitung 14 zur Sensoreinheit 11 erzeugt wird. Zieht man in der Look-Up-Tabelle 28 eine Gerade 33 bei 50 ml/min, erkennt man auf den ersten Blick, bei welcher Betriebsspannung der Fluidfördereinheit 24 welcher Gasdruck resultiert. Mittels der Einstelleinheit 26 kann dieser Vorgang automatisiert in das vorgeschlagene Verfahren implementiert werden.

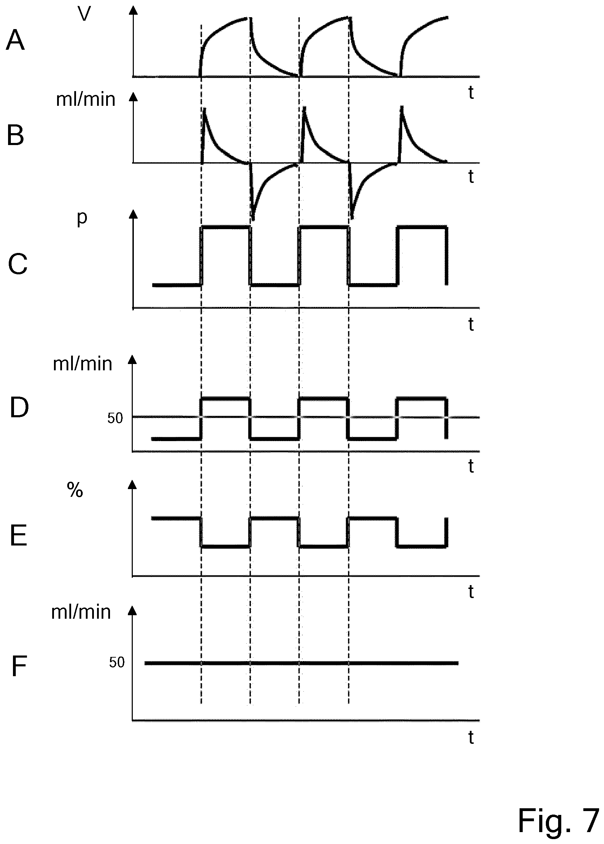

Fig. 7 zeigt Kennliniendiagramme zum Erläutern eines adaptiven Einstellens einer Fluidfördereinheit 24 während einer druckgeführten Beatmung durch das Medizingerät 12. In Kennliniendiagramm A ist das Volumen über die Zeit aufgetragen. In Kennliniendiagramm B ist der zugehörige Volumenstrom über die Zeit aufgetragen. In Kennliniendiagramm C ist der zugehörige Druck über die Zeit aufgetragen. In Kennliniendiagramm D ist ein sich verändernder Volumenstrom für den Fall gezeigt, in welchem die Fluidfördereinheit 24 gleichmäßig betrieben wird. Kennliniendiagramm E zeigt die sich ändernde Betriebsleistung der Fluidfördereinheit 24 über die Zeit, die aus dem vorstehend beschriebenen, adaptiven Einstellen der Fluidfördereinheit 24 resultiert. In Kennliniendiagramm F ist der gleichmäßige Volumenstrom gezeigt, der durch das adaptive Einstellen der Fluidfördereinheit 24 erzeugt wird.

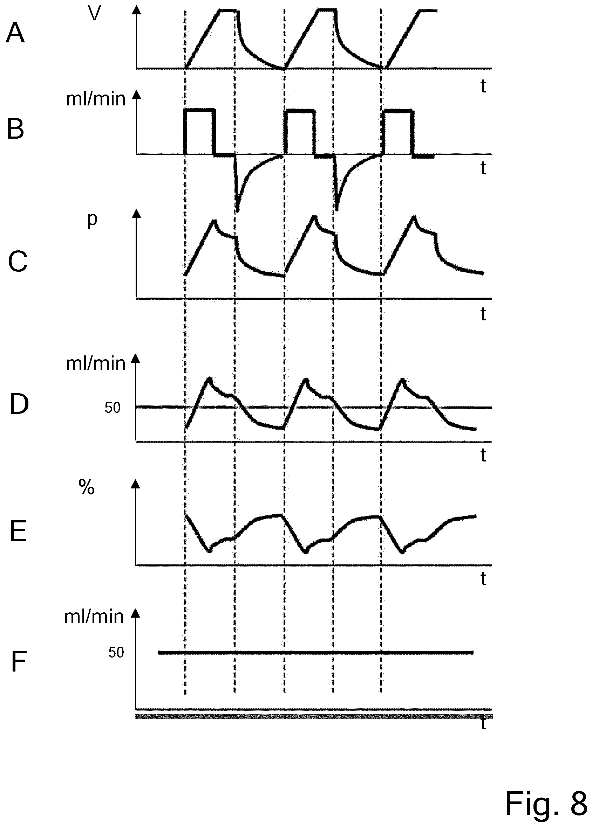

Fig. 8 zeigt Kennliniendiagramme zum Erläutern eines adaptiven Einstellens einer Fluidfördereinheit während einer volumengeführten Beatmung durch ein erfindungsgemäßes Medizingerät. In Kennliniendiagramm A ist das Volumen über die Zeit aufgetragen. In Kennliniendiagramm B ist der zugehörige Volumenstrom über die Zeit aufgetragen. In Kennliniendiagramm C ist der zugehörige Druck über die Zeit aufgetragen. In Kennliniendiagramm D ist ein sich verändernder Volumenstrom für den Fall gezeigt, in welchem die Fluidfördereinheit 24 gleichmäßig betrieben wird. Kennliniendiagramm E zeigt die sich ändernde Betriebsleistung der Fluidfördereinheit 24 über die Zeit, die aus dem vorstehend beschriebenen, adaptiven Einstellen der Fluidfördereinheit 24 resultiert. In Kennliniendiagramm F ist der gleichmäßige Volumenstrom gezeigt, der durch das adaptive Einstellen der Fluidfördereinheit 24 erzeugt wird. Zum Ermitteln der Kohlenstoffdioxidkonzentration im Messgas während der volumengeführten Beatmung der Person durch das Medizingerät 12 wird die Fluidfördereinheit 24, zum Erzeugen des gleichmäßigen Volumenstroms in der Abzweigungsleitung 14 zur Sensoreinheit 11, dabei unter Verwendung des aus der volumengeführten Beatmung der Person 13 resultierenden Atemwegsdrucks in der Hauptleitung 15 adaptiv eingestellt.

Die Erfindung lässt neben den dargestellten Ausführungsformen weitere Gestaltungsgrundsätze zu. D.h., die Erfindung soll nicht auf die mit Bezug auf die Figuren erläuterten Ausführungsbeispiele beschränkt betrachtet werden. Die Erfindung wird durch die nachstehenden Ansprüche beschränkt. So kann bei einem vorstehend beschriebenen Verfahren die Fluidfördereinheit 24, zum Erzeugen eines gleichmäßigen Volumenstroms und/oder eines gleichmäßigen Gasdrucks des Messgases in der Abzweigungsleitung 14 zur Sensoreinheit 11, ausschließlich unter Berücksichtigung eines Atemwegsdrucks während einer inspiratorischen Phase der Beatmung in der Hauptleitung 15 oder ausschließlich unter Berücksichtigung eines Atemwegsdrucks während einer exspiratorischen Phase der Beatmung in der Hauptleitung 15 adaptiv eingestellt werden. Außerdem kann bei einem vorstehend beschriebenen Verfahren die Fluidfördereinheit 24, zum Erzeugen eines gleichmäßigen Volumenstroms und/oder eines gleichmäßigen Gasdrucks des Messgases in der Abzweigungsleitung 14 zur Sensoreinheit 11, während einer exspiratorischen Phase der Beatmung mit gleichbleibender Leistung betrieben wird und während einer inspiratorischen Phase der Beatmung unter Berücksichtigung des Atemwegsdrucks während der inspiratorischen Phase der Beatmung adaptiv eingestellt werden. Darüber hinaus kann die Fluidfördereinheit 24, zum Erzeugen eines gleichmäßigen Volumenstroms und/oder eines gleichmäßigen Gasdrucks des Messgases in der Abzweigungsleitung 14 zur Sensoreinheit 11, während einer exspiratorischen Phase der Beatmung unter Berücksichtigung des Atemwegsdrucks während der exspiratorischen Phase der Beatmung adaptiv eingestellt werden und während einer inspiratorischen Phase der Beatmung deaktiviert werden.

### Bezugszeichenliste

- 10: Ermittlungsvorrichtung
- 11: Sensoreinheit
- 12: Medizingerät
- 13: Person
- 14: Abzweigungsleitung
- 15: Hauptleitung
- 16: HME-Filter
- 17: HME-Filter
- 20: Atemmaske
- 21: Inspirationsgasleitungsabschnitt
- 22: Gesamtgasleitungsabschnitt
- 23: Exspirationsgasleitungsabschnitt
- 24: Fluidfördereinheit
- 25: Exspirationsventil
- 26: Einstelleinheit
- 27: Atemwegsdruck-Sensor
- 28: Look-Up-Tabelle
- 29: Computerprogrammprodukt
- 30: Speichermittel
- 31: Ventilanschluss
- 32: Hauptpumpe
- 33: Gerade

## Patentansprüche

1. Verfahren zum Ermitteln einer Kohlenstoffdioxidkonzentration in Messgas, aufweisend die Schritte:
- Abzweigen des Messgases aus einer Hauptleitung (15) eines Medizingerätes (12) während einer inspiratorischen Phase einer am Medizingerät (12) angeschlossenen Person (13) als Inspirationsgas und während einer exspiratorischen Phase der Person (13) als Exspirationsgas zu einer Sensoreinheit (11) durch eine Abzweigungsleitung (14),
- Fördern des Messgases aus der Hauptleitung (15) durch die Abzweigungsleitung (14) zur Sensoreinheit (11) mittels einer Fluidfördereinheit (24),
- adaptives Einstellen der Fluidfördereinheit (24) unter Berücksichtigung eines Atemwegsdrucks in der Hauptleitung (15) zum Erzeugen eines während der inspiratorischen Phase und der exspiratorischen Phase gleichmäßigen Volumenstroms und/oder Gasdrucks des Messgases in der Abzweigungsleitung (14) zur Sensoreinheit (11), und
- Ermitteln der Kohlenstoffdioxidkonzentration im Messgas mittels der Sensoreinheit (11),
**dadurch gekennzeichnet, dass**
die Fluidfördereinheit (24) zum Fördern des Messgases aus der Hauptleitung (15) durch die Abzweigungsleitung (14) zur Sensoreinheit (11) eine Piezopumpe aufweist und eine Betriebsspannung der Piezopumpe, zum Erzeugen eines gleichmäßigen Volumenstroms und/oder Gasdrucks des Messgases in der Abzweigungsleitung (14) zur Sensoreinheit (11), unter Berücksichtigung des Atemwegsdrucks sowie unter Verwendung einer Look-Up-Tabelle (28) adaptiv eingestellt wird.

2. Verfahren nach Anspruch 1,
**dadurch gekennzeichnet, dass**
der Atemwegsdruck in der Hauptleitung (15) durch einen Atemwegsdruck-Sensor (27) gemessen wird und die Fluidfördereinheit (24), zum Erzeugen eines gleichmäßigen Volumenstroms und/oder Gasdrucks des Messgases in der Abzweigungsleitung (14) zur Sensoreinheit (11), unter Verwendung des gemessenen Atemwegsdrucks in der Hauptleitung (15) adaptiv eingestellt wird.

3. Verfahren nach einem der voranstehenden Ansprüche zum Ermitteln der Kohlenstoffdioxidkonzentration im Messgas während einer druckgeführten Beatmung der Person (13) durch das Medizingerät (12), wobei die Fluidfördereinheit (24), zum Erzeugen eines gleichmäßigen Volumenstroms und/oder Gasdrucks des Messgases in der Abzweigungsleitung (14) zur Sensoreinheit (11), unter Verwendung eines durch die druckgeführte Beatmung der Person (13) eingestellten Atemwegsdrucks in der Hauptleitung (15) adaptiv eingestellt wird.

4. Verfahren nach einem der Ansprüche 1 bis 2 zum Ermitteln der Kohlenstoffdioxidkonzentration im Messgas während einer volumengeführten Beatmung der Person (13) durch das Medizingerät (12), wobei die Fluidfördereinheit (24), zum Erzeugen eines gleichmäßigen Volumenstroms und/oder Gasdrucks des Messgases in der Abzweigungsleitung (14) zur Sensoreinheit (11), unter Verwendung eines aus der volumengeführten Beatmung der Person (13) resultierenden Atemwegsdrucks in der Hauptleitung (15) adaptiv eingestellt wird.

5. Verfahren nach einem der voranstehenden Ansprüche,
**dadurch gekennzeichnet, dass**
die Fluidfördereinheit (24), zum Erzeugen eines gleichmäßigen Volumenstroms und/oder Gasdrucks des Messgases in der Abzweigungsleitung (14) zur Sensoreinheit (11), ausschließlich unter Berücksichtigung eines Atemwegsdrucks während einer inspiratorischen Phase der Beatmung in der Hauptleitung (15) oder ausschließlich unter Berücksichtigung eines Atemwegsdrucks während einer exspiratorischen Phase der Beatmung in der Hauptleitung (15) adaptiv eingestellt wird.

6. Verfahren nach einem der voranstehenden Ansprüche,
**dadurch gekennzeichnet, dass**
die Fluidfördereinheit (24), zum Erzeugen eines gleichmäßigen Volumenstroms und/oder Gasdrucks des Messgases in der Abzweigungsleitung (14) zur Sensoreinheit (11), während einer exspiratorischen Phase der Beatmung mit gleichbleibender Leistung betrieben wird und während einer inspiratorischen Phase der Beatmung unter Berücksichtigung des Atemwegsdrucks während der inspiratorischen Phase der Beatmung adaptiv eingestellt wird.

7. Verfahren nach einem der voranstehenden Ansprüche,
**dadurch gekennzeichnet, dass**
die Fluidfördereinheit (24), zum Erzeugen eines gleichmäßigen Volumenstroms und/oder Gasdrucks des Messgases in der Abzweigungsleitung (14) zur Sensoreinheit (11), während einer exspiratorischen Phase der Beatmung unter Berücksichtigung des Atemwegsdrucks während der exspiratorischen Phase der Beatmung adaptiv eingestellt wird und während einer inspiratorischen Phase der Beatmung deaktiviert wird.

8. Ermittlungsvorrichtung (10) zum Ermitteln einer Kohlenstoffdioxidkonzentration in Messgas aus einem Medizingerät (12) während einer Beatmung einer Person (13) durch das Medizingerät (12), aufweisend:
- eine Sensoreinheit (11) zum Ermitteln der Kohlenstoffdioxidkonzentration im Messgas,
- eine Abzweigungsleitung (14) zum Abzweigen des Messgases aus einer Hauptleitung (15) des Medizingerätes (12) während einer inspiratorischen Phase der Person (13) als Inspirationsgas und während einer exspiratorischen Phase der Person (13) als Exspirationsgas zur Sensoreinheit (11),
- eine Fluidfördereinheit (24) mit einer Piezopumpe zum Fördern des Messgases aus der Hauptleitung (15) durch die Abzweigungsleitung (14) zur Sensoreinheit (11), und
- eine Einstelleinheit (26), die zum adaptiven Einstellen der Piezopumpe unter Berücksichtigung eines Atemwegsdrucks in der Hauptleitung (15) sowie unter Verwendung einer Look-Up-Tabelle (28), zum Erzeugen eines während der inspiratorischen Phase und der exspiratorischen Phase gleichmäßigen Volumenstroms und/oder Gasdrucks des Messgases in der Abzweigungsleitung (14) zur Sensoreinheit (11), konfiguriert und ausgestaltet ist.

9. Ermittlungsvorrichtung (10) nach Anspruch 8,
**gekennzeichnet durch**
einen Atemwegsdruck-Sensor (27) zum Messen des Atemwegsdrucks in der Hauptleitung (15), wobei die Einstelleinheit (26) zum adaptiven Einstellen der Fluidfördereinheit (24) unter Berücksichtigung des gemessenen Atemwegsdrucks in der Hauptleitung (15), zum Erzeugen eines gleichmäßigen Volumenstroms und/oder Gasdrucks des Messgases in der Abzweigungsleitung (14) zur Sensoreinheit (11), konfiguriert und ausgestaltet ist.

10. Ermittlungsvorrichtung (10) nach einem der Ansprüche 8 bis 9 zum Ermitteln einer Kohlenstoffdioxidkonzentration in Messgas aus einem Medizingerät (12) während einer druckgeführten Beatmung einer Person (13) durch das Medizingerät (12),
**dadurch gekennzeichnet, dass**
die Einstelleinheit (26) zum adaptiven Einstellen der Fluidfördereinheit (24) unter Verwendung eines durch die druckgeführte Beatmung der Person eingestellten Atemwegsdrucks in der Hauptleitung (15) konfiguriert ist.

11. Ermittlungsvorrichtung (10) nach einem der Ansprüche 8 bis 10 zum Ermitteln einer Kohlenstoffdioxidkonzentration in Messgas aus einem Medizingerät (12) während einer volumengeführten Beatmung einer Person (13) durch das Medizingerät (12),
**dadurch gekennzeichnet, dass**
die Einstelleinheit (26) zum adaptiven Einstellen der Fluidfördereinheit (24) unter Verwendung eines aus der volumengeführten Beatmung der Person resultierenden Atemwegsdrucks in der Hauptleitung (15) konfiguriert ist.

12. Medizingerät (12) zum Beatmen einer Person (13), aufweisend eine Hauptleitung (15) zum Leiten von Inspirationsgas und zum Leiten von Exspirationsgas, und eine Ermittlungsvorrichtung (10) nach einem der Ansprüche 8 bis 11 zum Ermitteln einer Kohlenstoffdioxidkonzentration in Messgas aus der Hauptleitung (15).

13. Medizingerät (12) nach Anspruch 12,
**dadurch gekennzeichnet, dass**
die Hauptleitung (15) einen Inspirationsgasleitungsabschnitt (21) zum Leiten eines Inspirationsgases und einen Gesamtgasleitungsabschnitt (22) zum Leiten des Inspirationsgases sowie eines Exspirationsgases aufweist, wobei die Abzweigungsleitung (14) zum Abzweigen des Messgases aus dem Gesamtgasleitungsabschnitt (22) ausgestaltet ist.

14. Computerprogrammprodukt (29), umfassend Befehle, die bei der Ausführung des Computerprogrammprodukts (29) durch einen Computer diesen veranlassen, das Verfahren nach einem der Ansprüche 1 bis 7 unter Verwendung einer Ermittlungsvorrichtung (10) nach einem der Ansprüche 8 bis 11 auszuführen.

15. Speichermittel (30) mit einem darauf gespeicherten Computerprogrammprodukt (29) nach Anspruch 14.

## Claims

1. Method for determining a carbon dioxide concentration in measuring gas, comprising the steps of:
- branching the measuring gas from a main line (15) of a medical device (12) during an inspiratory phase of a person (13) connected to the medical device (12) as inspiration gas, and during an expiratory phase of the person (13) as expiration gas to a sensor unit (11) through a branch line (14),
- conveying the measuring gas from the main line (15) through the branch line (14) to the sensor unit (11) by means of a fluid conveying unit (24),
- adaptively adjusting the fluid conveying unit (24), taking into account a respiratory tract pressure in the main line (15) for generating a uniform flow rate and/or gas pressure of the measuring gas in the branch line (14) to the sensor unit (11) during the inspiratory phase and the expiratory phase, and
- determining the carbon dioxide concentration in the measuring gas by means of the sensor unit (11),
**characterized in that**
the fluid conveying unit (24) for conveying the measuring gas from the main line (15) through the branch line (14) to the sensor unit (11) comprises a piezo pump, and an operating voltage of the piezo pump, for generating a uniform flow rate and/or gas pressure of the measuring gas in the branch line (14) to the sensor unit (11), is adaptively adjusted, taking into account the respiratory tract pressure and using a look-up table (28).

2. Method according to claim 1,
**characterized in that**
the respiratory tract pressure in the main line (15) is measured by a respiratory tract pressure sensor (27), and the fluid conveying unit (24), for generating a uniform flow rate and/or gas pressure of the measurement gas in the branch line (14) to the sensor unit (11), is adaptively adjusted using the measured respiratory tract pressure in the main line (15).

3. Method according to either of the preceding claims for determining the carbon dioxide concentration in measuring gas during pressure-controlled ventilation of the person (13) by the medical device (12), wherein the fluid conveying unit (24), for generating a uniform flow rate and/or gas pressure of the measuring gas in the branch line (14) to the sensor unit (11), is adaptively adjusted using a respiratory tract pressure in the main line (15) adjusted by the pressure-controlled ventilation of the person (13).

4. Method according to either of claims 1 to 2 for determining the carbon dioxide concentration in the measuring gas during volume-controlled ventilation of the person (13) by the medical device (12), wherein the fluid conveying unit (24), for generating a uniform flow rate and/or gas pressure of the measurement gas in the branch line (14) to the sensor unit (11), is adaptively adjusted using a respiratory tract pressure in the main line (15) resulting from the volume-controlled ventilation of the person (13).

5. Method according to any of the preceding claims,
**characterized in that**
the fluid conveying unit (24), for generating a uniform flow rate and/or gas pressure of the measuring gas in the branch line (14) to the sensor unit (11), is adaptively adjusted, exclusively taking into account a respiratory tract pressure during an inspiratory phase of the ventilation in the main line (15) or exclusively taking into account a respiratory tract pressure during an expiratory phase of the ventilation in the main line (15).

6. Method according to any of the preceding claims,
**characterized in that**
the fluid conveying unit (24), for generating a uniform flow rate and/or gas pressure of the measurement gas in the branch line (14) to the sensor unit (11), is operated at constant power during an expiratory phase of the ventilation, and adaptively adjusted during an inspiratory phase of the ventilation, taking into account the respiratory tract pressure during the inspiratory phase of the ventilation.

7. Method according to any of the preceding claims,
**characterized in that**
the fluid conveying unit (24), for generating a uniform flow rate and/or gas pressure of the measurement gas in the branch line (14) to the sensor unit (11), is adaptively adjusted during an expiratory phase of the ventilation, taking into account the respiratory tract pressure during the expiratory phase of the ventilation, and is deactivated during an inspiratory phase of the ventilation.

8. Determining apparatus (10) for determining a carbon dioxide concentration in measuring gas from a medical device (12) during ventilation of a person (13) by the medical device (12), comprising:
- a sensor unit (11) for determining the carbon dioxide concentration in the measuring gas,
- a branch line (14) for branching the measuring gas from a main line (15) of the medical device (12) during an inspiratory phase of the person (13) as inspiration gas and during an expiratory phase of the person (13) as expiration gas to the sensor unit (11),
- a fluid conveying unit (24) having a piezo pump for conveying the measuring gas from the main line (15) through the branch line (14) to the sensor unit (11), and
- an adjustment unit (26) which is configured and designed to adaptively adjust the piezo pump, taking into account a respiratory tract pressure in the main line (15) and using a look-up table (28), for generating a flow rate, which is uniform during the inspiratory phase and the expiratory phase, and/or gas pressure of the measurement gas in the branch line (14) to the sensor unit (11).

9. Determining apparatus (10) according to claim 8,
**characterized by**
a respiratory tract pressure sensor (27) for measuring the respiratory tract pressure in the main line (15), the adjustment unit (26) being configured and designed to adaptively adjust the fluid conveying unit (24), taking into account the measured respiratory tract pressure in the main line (15), in order to generate a uniform flow rate and/or gas pressure of the measurement gas in the branch line (14) to the sensor unit (11).

10. Determining apparatus (10) according to either of claims 8 to 9 for determining a carbon dioxide concentration in measuring gas from a medical device (12) during pressure-controlled ventilation of a person (13) by the medical device (12),
**characterized in that**
the adjustment unit (26) is configured to adaptively adjust the fluid conveying unit (24) using a respiratory tract pressure in the main line (15) adjusted by the pressure-controlled ventilation of the person.

11. Determining apparatus (10) according to any of claims 8 to 10 for determining a carbon dioxide concentration in measuring gas from a medical device (12) during volume-controlled ventilation of a person (13) by the medical device (12),
**characterized in that**
the adjustment unit (26) is configured to adaptively adjust the fluid conveying unit (24) using a respiratory tract pressure in the main line (15) resulting from the volume-controlled ventilation of the person.

12. Medical device (12) for ventilating a person (13), comprising a main line (15) for conducting inspiration gas and for conducting expiration gas, and a determining apparatus (10) according to any of claims 8 to 11 for determining a carbon dioxide concentration in measuring gas from the main line (15).

13. Medical device (12) according to claim 12,
**characterized in that**
the main line (15) comprises an inspiration gas line portion (21) for conducting an inspiration gas and a total gas line portion (22) for conducting the inspiration gas and an expiration gas, the branch line (14) being designed to branch the measuring gas from the total gas line portion (22).

14. Computer program product (29) comprising instructions which, when the computer program product (29) is executed by a computer, cause the computer to carry out the method according to any of claims 1 to 7 using a determining apparatus (10) according to any of claims 8 to 11.

15. Storage means (30) having a computer program product (29) according to claim 14 stored thereon.

## Revendications

1. Procédé permettant de déterminer une concentration de dioxyde de carbone dans un gaz de mesure, présentant les étapes suivantes :
- dérivation du gaz de mesure à partir d'une conduite principale (15) d'un appareil médical (12) pendant une phase inspiratoire d'une personne (13) reliée à l'appareil médical (12), en tant que gaz d'inspiration, et pendant une phase d'expiration de la personne (13) en tant que gaz d'expiration vers une unité de détection (11) à travers une conduite de dérivation (14),
- transport du gaz de mesure à partir de la conduite principale (15) vers l'unité de détection (11) à travers la conduite de dérivation (14), au moyen d'une unité de transport de fluide (24),
- réglage adaptatif de l'unité de transport de fluide (24) en tenant compte d'une pression des voies respiratoires dans la conduite principale (15) pour la génération d'un débit volumique et/ou une pression de gaz du gaz de mesure dans la conduite de dérivation (14) vers l'unité de détection (11), lequel débit volumique et/ou laquelle pression de gaz sont uniformes pendant la phase d'inspiration et la phase d'expiration, et
- détermination de la concentration de dioxyde de carbone dans le gaz de mesure au moyen de l'unité de détection (11),
**caractérisé en ce que**
l'unité de transport de fluide (24) présente une pompe piézo pour le de transport du gaz de mesure à partir de la conduite principale (15) vers l'unité de détection (11) à travers la conduite de dérivation (14), et une tension de fonctionnement de la pompe piézo est réglée de manière adaptative pour la génération d'un débit volumique et/ou une pression de gaz uniformes du gaz de mesure dans la conduite de dérivation (14) vers l'unité de détection (11), en tenant compte de la pression des voies respiratoires et à l'aide d'un tableau de correspondance (28).

2. Procédé selon la revendication 1,
**caractérisé en ce que**
la pression des voies respiratoires dans la conduite principale (15) est mesurée par un capteur de pression des voies respiratoires (27) et l'unité de transport de fluide (24) est réglée de manière adaptative pour la génération d'un débit volumique et/ou une pression de gaz uniformes du gaz de mesure dans la conduite de dérivation (14) vers l'unité de détection (11), à l'aide de la pression des voies respiratoires mesurée dans la conduite principale (15).

3. Procédé selon l'une des revendications précédentes, permettant de déterminer la concentration de dioxyde de carbone dans le gaz de mesure pendant une respiration sous pression de la personne (13) à travers l'appareil médical (12), dans lequel l'unité de transport de fluide (24) est réglée de manière adaptative pour la génération un débit volumique et/ou une pression de gaz uniformes du gaz de mesure dans la conduite de dérivation (14) vers l'unité de détection (11), à l'aide d'une pression des voies respiratoires réglée par la respiration sous pression de la personne (13) dans la conduite principale (15).

4. Procédé selon l'une des revendications 1 à 2, permettant de déterminer la concentration de dioxyde de carbone dans le gaz de mesure pendant une respiration à volume contrôlé de la personne (13) à travers l'appareil médical (12), dans lequel l'unité de transport de fluide (24) est réglée de manière adaptative pour la génération d'un débit volumique et/ou une pression de gaz uniformes du gaz de mesure dans la conduite de dérivation (14) vers l'unité de détection (11), à l'aide d'une pression des voies respiratoires résultant de la respiration à volume contrôlé de la personne (13) dans la conduite principale (15).

5. Procédé selon l'une des revendications précédentes,
**caractérisé en ce que**
pour la génération d'un débit volumique et/ou une pression de gaz uniformes du gaz de mesure dans la conduite de dérivation (14) vers l'unité de détection (11), l'unité de transport de fluide (24) est réglée de manière adaptative en tenant compte uniquement d'une pression des voies respiratoires pendant une phase d'inspiration de la respiration dans la conduite principale (15) ou en tenant compte uniquement d'une pression des voies respiratoires pendant une phase d'expiration de la respiration dans la conduite principale (15).

6. Procédé selon l'une des revendications précédentes,
**caractérisé en ce que**
pour la génération d'un débit volumique et/ou une pression de gaz uniformes du gaz de mesure dans la conduite de dérivation (14) vers l'unité de détection (11), l'unité de transport de fluide (24) fonctionne avec une puissance constante pendant une phase d'expiration de la respiration, et est réglée de manière adaptative pendant une phase d'inspiration de la respiration en tenant compte de la pression des voies respiratoires pendant la phase d'inspiration de la respiration.

7. Procédé selon l'une des revendications précédentes,
**caractérisé en ce que**
pour la génération d'un débit volumique et/ou une pression de gaz uniformes du gaz de mesure dans la conduite de dérivation (14) vers l'unité de détection (11), l'unité de transport de fluide (24) est réglée de manière adaptative pendant une phase d'expiration de la respiration en tenant compte de la pression des voies respiratoires pendant la phase d'expiration de la respiration, et est désactivée pendant une phase d'inspiration de la respiration.

8. Dispositif de détermination (10) permettant de déterminer une concentration de dioxyde de carbone d'un gaz de mesure à partir d'un appareil médical (12) pendant une respiration d'une personne (13) à travers l'appareil médical (12), présentant :
- une unité de détection (11) permettant de déterminer une concentration de dioxyde de carbone dans le gaz de mesure,
- une conduite de dérivation (14) permettant de dériver, vers l'unité de détection (11), le gaz de mesure à partir d'une conduite principale (15) d'un appareil médical (12) pendant une phase d'inspiration de la personne (13) en tant que gaz d'inspiration, et pendant une phase d'expiration de la personne (13) en tant que gaz d'expiration,
- une unité de transport de fluide (24) comportant une pompe piézo permettant de transporter le gaz de mesure à partir de la conduite principale (15) vers l'unité de détection (11) à travers la conduite de dérivation (14), et
- une unité de réglage (26) qui est configurée et conçue pour le réglage adaptif de la pompe piézo en tenant compte d'une pression des voies respiratoires dans la conduite principale (15) et à l'aide d'un tableau de correspondance (28) pour la génération d'un débit volumique et/ou une pression de gaz du gaz de mesure dans la conduite de dérivation (14) vers l'unité de détection (11), lequel débit volumique et/ou laquelle pression de gaz sont uniformes pendant la phase d'inspiration et la phase d'expiration.

9. Dispositif de détermination (10) selon la revendication 8,
**caractérisé par**
un capteur de pression des voies respiratoires (27) permettant de mesurer la pression des voies respiratoires dans la conduite principale (15), dans lequel l'unité de réglage (26) est configurée et conçue pour le réglage adaptif de l'unité de transport de fluide (24) en tenant compte de la pression des voies respiratoires mesurée dans la conduite principale (15), pour la génération d'un débit volumique et/ou une pression de gaz uniformes du gaz de mesure dans la conduite de dérivation (14) vers l'unité de détection (11).

10. Dispositif de détermination (10) selon l'une des revendications 8 à 9, permettant de déterminer concentration de dioxyde de carbone d'un gaz de mesure à partir d'un appareil médical (12) pendant une respiration sous pression d'une personne (13) à travers l'appareil médical (12),
**caractérisé en ce que**
l'unité de réglage (26) est configurée pour le réglage adaptif de l'unité de transport de fluide (24) à l'aide d'une pression des voies respiratoires dans la conduite principale (15), laquelle pression des voies respiratoires est réglée par la respiration sous pression de la personne.

11. Dispositif de détermination (10) selon l'une des revendications 8 à 10, permettant de déterminer concentration de dioxyde de carbone d'un gaz de mesure à partir d'un appareil médical (12) pendant une respiration à volume contrôlé d'une personne (13) à travers l'appareil médical (12),
**caractérisé en ce que**
l'unité de réglage (26) est configurée pour le réglage adaptif de l'unité de transport de fluide (24) à l'aide d'une pression des voies respiratoires dans la conduite principale (15), laquelle pression des voies respiratoires résulte de la respiration à volume contrôlé de la personne.

12. Appareil médical (12) permettant de respirer une personne (13), présentant une conduite principale (15) pour la conduite d'un gaz d'inspiration et la conduite d'un gaz d'expiration, et un dispositif de détermination (10) selon l'une des revendications 8 à 11, permettant de déterminer une concentration en oxyde de carbone dans un gaz de mesure à partir de la conduite principale (15).

13. Appareil médical (12) selon la revendication 12,
**caractérisé en ce que**
la conduite principale (15) présente une section de conduite de gaz d'inspiration (21) pour la conduite d'un gaz d'inspiration et une section de conduite de gaz d'ensemble (22) pour la conduite du gaz d'inspiration et d'un gaz d'expiration, dans lequel la conduite de dérivation (14) est conçue pour la dérivation du gaz de mesure à partir de la section de conduite de gaz d'ensemble (22).

14. Produit de programme informatique (29) comprenant des instructions qui, lorsque le produit de programme informatique (29) est exécuté par un ordinateur, amènent celui-ci à exécuter le procédé selon l'une des revendications 1 à 7 à l'aide d'un dispositif de détermination (10) selon l'une des revendications 8 à 11.

15. Moyen de stockage (30) comportant un produit de programme informatique (29) selon la revendication 14 stocké sur celui-ci.
